# EUROPEAN PATENT SPECIFICATION

(11) **EP 2 981 254 B1**
(45) Date of publication and mention of the grant of the patent: **10.10.2018**
(21) Application number: 14779926.6
(22) Date of filing: 07.04.2014
(51) Int. Cl.: A61K 31/12, A61K 31/192, A61K 31/222, A61K 31/353, A61K 31/216, A61K 31/235, A61P 35/00

(54) **THERAPEUTIC COMPOSITIONS COMPRISING EXTRACTS OF PROPOLIS AND USES THEREOF FOR THE TREATMENT OF GASTRIC CANCER**
THERAPEUTISCHE ZUSAMMENSETZUNGEN MIT EXTRAKTEN AUS PROPOLIS UND VERWENDUNGEN DAVON ZUR BEHANDLUNG VON MAGENKREBS
COMPOSITIONS THÉRAPEUTIQUES COMPRENANT DES EXTRAITS DE PROPOLIS ET LEURS UTILISATIONS POUR LE TRAITEMENT DE CANCERS GASTRIQUES

(30) Priority: 05.04.2013 NZ 60910613
(43) Date of publication of application: 10.02.2016
(73) Proprietor: Manuka Health New Zealand Limited, Mt Wellington, Auckland 1072 (NZ)
(72) Inventor: PAUL, Kerry, Mt Wellington, Auckland 1072 (NZ); CATCHPOLE, Owen, Mt Wellington, Auckland 1072 (NZ); MITCHELL, Kevin, Mt Wellington, Auckland 1072 (NZ)
(74) Representative: Casley, Christopher Stuart
(86) International application number: PCT/NZ2014/000059
(87) International publication number: WO 2014/163512

(56) References cited:
- WO-A1-2011/156889
- WO-A1-2014/163513
- WO-A2-02/47615
- WO-A2-2013/022740
- JP-A- 2003 119 169
- US-A1- 2002 188 021
- US-A1- 2006 135 585
- Aimaiti Mutalifu: "The Effects of Propolis flavonoid Pinobanksin-3-acetate on Cell Proliferation, Apoptosis and Gene Expression of Human Colorectal Cancer Cell Lines", Master's Thesis, 1 January 2012 (2012-01-01), XP055291910, Retrieved from the Internet: URL:http://www.topresearch.org/showinfo-17 1-827212-0.html [retrieved on 2016-07-28]
- Li Feng ET AL: "Cytotoxicity of Constituents from Mexican Propolis Against a Panel of Six Different Cancer Cell Lines", Natural product communications, 1 October 2010 (2010-10-01), pages 1601-1606, XP055291824, Retrieved from the Internet: URL:https://www.researchgate.net/profile/S uresh_Awale/publication/49648580_Cytotoxic ity_of_Constituents_from_Mexican_Propolis_ Against_a_Panel_of_Six_Different_Cancer_Ce ll_Lines/links/0c96053b49725d0b56000000.pd f?origin=publication_detail [retrieved on 2016-07-28]
- TEPY USIA ET AL: "Constituents of Chinese Propolis and Their Antiproliferative Activities", JOURNAL OF NATURAL PRODUCTS., vol. 65, no. 5, 1 May 2002 (2002-05-01), pages 673-676, XP055285565, US ISSN: 0163-3864, DOI: 10.1021/np010486c
- KUMAR M A SURESH ET AL: "Pinocembrin triggers bax-dependent mitochondrial apoptosis in colon cancer cells", MOLECULAR CARCINOGENESIS, vol. 46, no. 3, March 2007 (2007-03), pages 231-241, XP002760349, ISSN: 0899-1987
- DATABASE WPI Week 201117 Thomson Scientific, London, GB; AN 2010-P99621 XP002760350, "Extraction of pinostrobin from medicinal plant, e.g. used in treating gastroduodenal ulcer, by extracting medicinal plants with ethanol, concentrating extract solution, and performing chromatographic separation via gel resin column", & CN 101 875 648 A (MA LINGYUAN) 3 November 2010 (2010-11-03)
- YOUNG LEE: "Flavanones inhibit the clonogenicity of HCT116 cololectal cancer cells", INTERNATIONAL JOURNAL OF MOLECULAR MEDICINE, 12 December 2011 (2011-12-12), pages 403-408, XP055285555, GR ISSN: 1107-3756, DOI: 10.3892/ijmm.2011.857
- RAO C V ET AL: "Chemoprevention of colon carcinogenesis by phenylethyl-3-methylcaffeate", CANCER RESEARCH, vol. 55, no. 11, 1 June 1995 (1995-06-01), pages 2310-2315, XP002728357, AMERICAN ASSOCIATION FOR CANCER RESEARCH, US ISSN: 0008-5472
- Chinthalapally Y Rao ET AL: "Inhibitory Effect of Caffeic Acid Esters on Azoxymethane-induced Biochemical Changes and Aberrant Crypt Foci Formation in Rat Colon", Cancer Research , 1 January 1993 (1993-01-01), pages 4182-4188, XP055322539, Retrieved from the Internet: URL:http://cancerres.aacrjournals.org/cont ent/canres/53/18/4182.full.pdf [retrieved on 2016-11-24]
- MICHAEL A. LEA ET AL: "Induction of histone acetylation and inhibition of growth by phenyl alkanoic acids and structurally related molecules", CANCER CHEMOTHERAPY AND PHARMACOLOGY., vol. 54, no. 1, 19 March 2004 (2004-03-19) , pages 57-63, XP055323037, BERLIN. ISSN: 0344-5704, DOI: 10.1007/s00280-004-0782-5
- ARJUN H BANSKOTA ET AL: "Antiproliferative activity of the Netherlands propolis and its active principles in cancer cell lines", JOURNAL OF ETHNOPHARMACOLOGY, vol. 80, no. 1, 1 January 2002 (2002-01-01), pages 67-73, XP008156193, ELSEVIER IRELAND LTD, IE ISSN: 0378-8741, DOI: 10.1016/S0378-8741(02)00022-3
- BOLLEDDULA JAYAPRAKASAM ET AL: "Impact of Alkyl Esters of Caffeic and Ferulic Acids on Tumor Cell Proliferation, Cyclooxygenase Enzyme, and Lipid Peroxidation", JOURNAL OF AGRICULTURAL AND FOOD CHEMISTRY, vol. 54, no. 15, 1 July 2006 (2006-07-01), pages 5375-5381, XP055026285, ISSN: 0021-8561, DOI: 10.1021/jf060899p
- XIA C N ET AL: "Synthesis of trans-caffeate analogues and their bioactivities against HIV-1 integrase and cancer cell lines", BIOORGANIC & MEDICINAL CHEMISTRY LETTERS, vol. 18, no. 24, 15 December 2008 (2008-12-15), pages 6553-6557, XP025679073, PERGAMON, AMSTERDAM, NL ISSN: 0960-894X, DOI: 10.1016/J.BMCL.2008.10.046 [retrieved on 2008-10-15]
- DATABASE WPI Week 200974 Thomson Scientific, London, GB; AN 2009-M79192 XP002764632, "Use of 3,4-dihydroxyphenylethyl caffeate in preparing antineoplastic medicine for treating tumor in digestive system, lung cancer, breast cancer, oophoroma, prostatic cancer, tumor in central nervous system, melanoma and leukemia", & CN 101 502 506 A (LIAN X) 12 August 2009 (2009-08-12)
- DATABASE WPI Week 201351 Thomson Scientific, London, GB; AN 2013-J48254 XP002764633, "Use of caffeic acid 3,4-dihydroxy-phenethyl ester for preparing antitumor medicinal composition for treating myeloma, preferably single and multiple myeloma, esophagus cancer and ear, nose and throat tumors e.g. nasal cancer", & CN 102 949 384 A (UNIV ZHEJIANG) 6 March 2013 (2013-03-06)
- DATABASE WPI Week 200879 Thomson Scientific, London, GB; AN 2008-N65708 XP002764634, "New caffeic acid derivatives used as angiogenesis inhibitor and tumor growth inhibitor and for preventing vascular diseases", -& KR 2008 0030938 A (UNIV SEOUL NAT IND FOUND) 7 April 2008 (2008-04-07)
- DATABASE WPI Week 201479 Thomson Scientific, London, GB; AN 2014-V39745 XP002764635, "New (Z)-phenethyl 3-(3,4-dimethoxyphenyl)acrylate derivatives useful for producing neuroprotective agent and antineoplastic drugs", & CN 104 016 862 A (UNIV PEKING) 3 September 2014 (2014-09-03)
- CATCHPOLE OWEN ET AL: "Antiproliferative activity of New Zealand propolis and phenolic compounds vs human colorectal adenocarcinoma cells", FITOTERAPIA, vol. 106, 9 September 2015 (2015-09-09), pages 167-174, XP029288110, ISSN: 0367-326X, DOI: 10.1016/J.FITOTE.2015.09.004
- OHTSUKI, T. ET AL.: 'Death receptor 5 promoter-enhancing compounds isolated from Catimbium speciosum and their enhancement effect on TRAIL-induced apoptosis' BIOORGANIC & MEDICINAL CHEMISTRY vol. 17, no. 18, 2009, pages 6748 - 6754, XP026545215
- WANG, ZHENRAN ET AL.: 'Alpinetin promotes Bax translocation, induces apoptosis through the mitochondrial pathway and arrests human gastric cancer cells at the G2/M phase' MOLECULAR MEDICINE REPORTS vol. 7, no. 3, March 2013, pages 915 - 920, XP055285552
- WOO, YOONKYUNG ET AL.: 'Flavanones inhibit the clonogenicity of HCT116 cololectal cancer cells' INTERNATIONAL JOURNAL OF MOLECULAR MEDICINE vol. 29, no. 3, 2012, pages 403 - 408, XP055285555
- HAN, CHUNHUA ET AL.: 'Inhibition of the Growth of Premalignant and Malignant Human Oral Cell Lines by Extracts and Components of Black Raspberries' NUTRITION AND CANCER vol. 51, no. 2, 2005, pages 207 - 217, XP008181279
- USIA, TEPY ET AL.: 'Constituents of Chinese Propolis and Their Antiproliferative Activities' JOURNAL OF NATURAL PRODUCTS vol. 65, no. 5, 2002, pages 673 - 676, XP055285565

## Description

### FIELD OF INVENTION

This invention relates to compositions for the treatment and prevention of gastrointestinal cancer, as defined in the claims. In particular, this disclosure relates to anti-gastrointestinal cancer compositions containing compounds derived from propolis, including anti-gastrointestinal cancer compositions comprising one or more propolis extracts enriched in one or more of these compounds. Particularly contemplated is the use of such compositions in the treatment or prevention of colorectal, throat and gastric cancers.

The invention relates to a compound of formula (II) or a composition comprising a therapeutically effective amount of said compound for use in the treatment or prevention of a gastric tumour or gastric cancer, as defined in the claims.

### BACKGROUND OF THE INVENTION

Colorectal cancer is reportedly the second and third most common cancer in women and men, respectively, from developed countries. Colorectal cancer is more prevalent in developed countries - the US, Australia, Europe, and New Zealand having the highest rates - with incidence being as much as 10 times greater than in developing countries. While surgery can be effective, early detection is critical to positive surgical outcomes. Other therapies are largely directed at life extension and palliative care, as the efficacy of current chemotherapies and radiotherapies in treating primary tumours, or metastases outside the lymph nodes is debated.

Throat cancer, also referred to as oesophaegeal cancer, pharyngeal cancer, or laryngeal cancer, encompasses tumours that develop in the tissues of the pharynx, nasopharynx, oropharynx, hypopharynx, larynx (voice box) or tonsils. Therapies for throat cancer include surgery, radiotherapy or chemotherapy. Treatment for throat cancer can damage the throat and may cause changes to the way a patient eats, breathes and sleeps.

Gastric or stomach cancer is the second most common cause of cancer-related death in the world. Diagnosis is often delayed because symptoms may not occur in the early stages of the disease. Surgery to remove the stomach (gastrectomy) is the only treatment that can cure gastric cancers. Chemotherapy and radiation therapy after surgery may improve the chance of a cure.

Bolleddula Jayaprakasam et al. (2006). Journal of Agricultural and Food Chemistry, 54, 5375-5381, describes the synthesis and testing of ferulic and caffeic acid esters.

Xia et al. (2008). Bioorganic & Medicinal Chemistry Letters, 18, 6553-6557, describes the synthesis of forty caffeate analogues and the testing of these analogues against HIV-1 integrase.

CN 101 502 506 A discloses preparation of anti-neoplastic medicine using 3,4-dihydroxyphenylethyl caffeate.

There is a need for anti-gastrointestinal cancer compositions, including those suitable for use in the treatment or prevention of colorectal cancer, gastric cancer or throat cancer and those which are able to support the maintenance of anti-gastrointestinal cancer activity or augment anti-gastrointestinal cancer activity against colorectal, gastric or throat cancers.

It is an object of the present invention to provide anti-gastrointestinal cancer compositions, including stable anti-gastrointestinal cancer compositions, for use in the treatment or prevention of gastric cancer or gastric tumours.

### SUMMARY OF THE INVENTION

Subject matter which is not encompassed by the by the scope of the claims does not form part of the present claimed invention.

In a first aspect the present invention relates to a compound of formula (II): 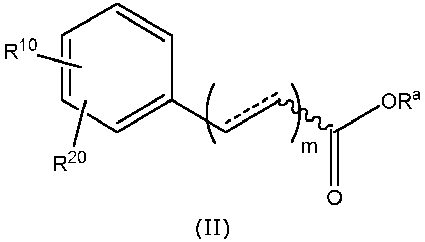 wherein:
R^{a} is C₂₋₆alkenyl or arylC₁₋₆alkyl;
m is 1 or 2; and
R¹⁰ and R²⁰ are each independently hydroxyl, or C₁₋₆alkoxy; and
provided that R^{a} is not arylC₁₋₆alkyl when R¹⁰ and R²⁰ are both hydroxyl,
for use in inhibiting gastrointestinal tumour formation, inhibiting gastrointestinal tumour growth, inhibiting gastrointestinal tumour metastasis or treating or preventing gastrointestinal cancer in a human subject; inducing apoptosis of one or more neoplastic gastrointestinal cells in a human subject; increasing the responsiveness of a human subject to a gastrointestinal cancer therapy; increasing the sensitivity of a gastrointestinal tumour in a human subject to a gastrointestinal cancer therapy; resensitising one or more gastrointestinal cancer cells in a human subject that are resistant to treatment; at least partially reversing the resistance of a neoplastic cell in a human subject suffering from gastrointestinal cancer to a gastrointestinal cancer therapy; reversing, wholly or in part, the resistance of a gastrointestinal cancer-burdened human patient to a gastrointestinal cancer therapy; or re-sensitising one or more tumours of a gastrointestinal cancer-burdened human patient which are, or are predicted to either be or become, resistant to treatment with a gastrointestinal cancer therapy to treatment with a gastrointestinal cancer therapy,
wherein the gastrointestinal tumour is a gastric tumour, and the gastrointestinal cancer is gastric cancer.
In a second aspect the present invention relates to a composition comprising a therapeutically effective amount of a compound of formula (II): 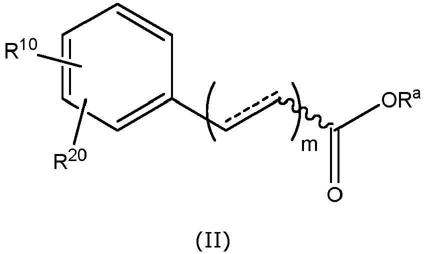 wherein:
R^{a} is C₂₋₆alkenyl or arylC₁₋₆alkyl;
m is 1 or 2; and
R¹⁰ and R²⁰ are each independently hydroxyl, or C₁₋₆alkoxy; and
provided that R^{a} is not arylC₁₋₆alkyl when R¹⁰ and R²⁰ are both hydroxyl,
for use in inhibiting gastrointestinal tumour formation, inhibiting gastrointestinal tumour growth, inhibiting gastrointestinal tumour metastasis or treating or preventing gastrointestinal cancer in a human subject; inducing apoptosis of one or more neoplastic gastrointestinal cells in a human subject; increasing the responsiveness of a human subject to a gastrointestinal cancer therapy; increasing the sensitivity of a gastrointestinal tumour in a human subject to a gastrointestinal cancer therapy; resensitising one or more gastrointestinal cancer cells in a human subject that are resistant to treatment; at least partially reversing the resistance of a neoplastic cell in a human subject suffering from gastrointestinal cancer to a gastrointestinal cancer therapy; reversing, wholly or in part, the resistance of a gastrointestinal cancer-burdened human patient to a gastrointestinal cancer therapy; or re-sensitising one or more tumours of a gastrointestinal cancer-burdened human patient which are, or are predicted to either be or become, resistant to treatment with a gastrointestinal cancer therapy to treatment with a gastrointestinal cancer therapy
wherein the gastrointestinal tumour is a gastric tumour and the gastrointestinal cancer is gastric cancer.

Described herein is a pharmaceutical composition comprising a therapeutically effective amount of a compound of formula (I) or (II): 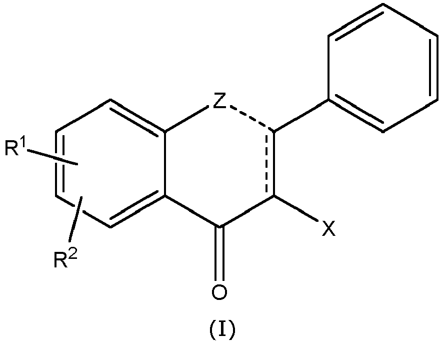 wherein:
Z is O or hydroxyl;
X is hydrogen, C₁₋₆alkylC(O)O, or C₁₋₆alkyl;
------ is a single bond when Z is O or absent when Z is hydroxyl;
-̅-̅-̅-̅-̅-̅ is a single bond or a double bond; and
R¹ and R² are each independently hydroxyl or C₁₋₆alkoxyl; and
provided that at least one of R¹ and R² is hydroxyl; and
provided that R¹ and R² are not both hydroxyl when X is H and -̅-̅-̅-̅-̅-̅ is a double bond; 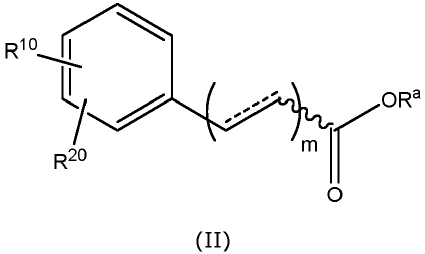 wherein:
R^{a} is hydrogen, C₂₋₆alkenyl, arylC₁₋₆alkyl, or arylC₂₋₆alkenyl;
m is 1 or 2; and
R¹⁰ and R²⁰ are each independently hydrogen, hydroxyl, or C₁₋₆alkoxy; and
provided that R^{a} is not arylC₁₋₆alkyl or arylC₂₋₆alkenyl when R¹⁰ and R²⁰ are both hydroxyl.
In one embodiment the compound of formula (I) is a compound of the formula (IA): 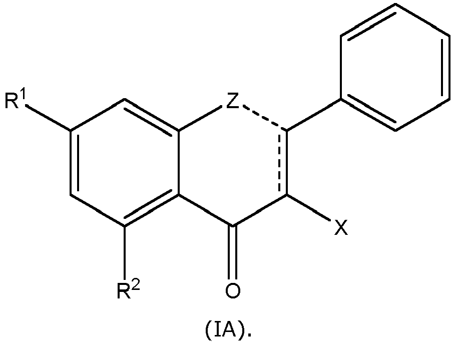 In one embodiment X is hydrogen, C₁₋₆alkylC(O)O, or C₂₋₅alkyl.
In one embodiment X is hydrogen or C₁₋₆alkylC(O)O.
In one embodiment the C₁₋₆alkylC(O)O is MeC(O)O.
In one embodiment R¹ and R² are each independently hydroxyl or C₁₋₆alkoxy.
In one embodiment R¹ is hydroxyl and R² is C₁₋₆alkoxy, R¹ is C₁₋₆alkoxy and R² is hydroxyl, or R¹ and R² are each hydroxyl.
In one embodiment the C₁₋₆alkoxy is OMe.
In one embodiment Z is O; ------ is a single bond; -̅-̅-̅-̅-̅-̅ is a single bond; X is C₁₋₆alkylC(O)O; and R¹ and R² are each hydroxyl.
In one embodiment Z is O and ------ is a single bond or Z is hydroxyl and ------ is absent; -̅-̅-̅-̅-̅-̅ is a double bond; X is hydrogen; and R¹ is hydroxyl and R² is C₁₋₆alkoxy or R¹ is C₁₋₆alkoxy and R² is hydroxyl.
In one embodiment R¹ is C₁₋₆alkoxy and R² is hydroxyl.
In one embodiment Z is O; and ------ is a single bond.
In one embodiment Z is hydroxyl; and ------ is absent.
In one embodiment the compound of formula (II) is a compound of the formula (IIA): 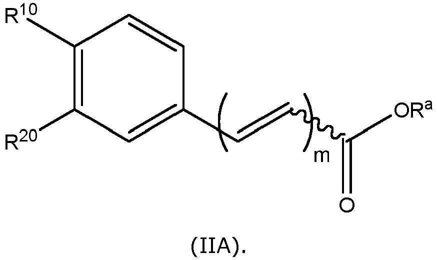 In one embodiment R¹⁰ and R²⁰ are each hydrogen, R¹⁰ and R²⁰ are each hydroxyl, R¹⁰ is hydroxyl and R²⁰ is C₁₋₆alkoxy, or R¹⁰ is C₁₋₆alkoxy and R²⁰ is hydroxyl.
In one embodiment the C₁₋₆alkoxy is OMe.
In one embodiment wherein R^{a} is hydrogen, C₂₋₆alkenyl, or arylC₁₋₆alkyl.
In one embodiment the C₂₋₆alkenyl is prenyl or isoprenyl.
In one embodiment the arylC₁₋₆alkyl is benzyl.
In one embodiment m is 2.
In one embodiment Ra is hydrogen and R¹⁰ and R²⁰ are each hydrogen.
In one embodiment m is 1.
In one embodiment R^{a} is C₂₋₆alkenyl or arylC₁₋₆alkyl; and R¹⁰ and R²⁰ are each hydroxyl, R¹⁰ is hydroxyl and R²⁰ is C₁₋₆alkoxy, or R¹⁰ is C₁₋₆alkoxy and R²⁰ is hydroxyl. In one embodiment R^{a} is C₂₋₆alkenyl; and R¹⁰ and R²⁰ are each hydroxyl.
In one embodiment Ra is arylC₁₋₆alkyl; R¹⁰ is hydroxyl and R²⁰ is C₁₋₆alkoxy, or R¹⁰ is C₁₋₆alkoxy and R²⁰ is hydroxyl.

Described herein is an anti-gastrointestinal cancer composition comprising at least one compound as defined herein, including a compound of formula (I) or (II), or a compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate.

For the avoidance of doubt, 5-phenylpenta-2,4-dienoic acid has formula (A), 3-methyl-3-butenyl caffeic acid has formula (B), 1,1-dimethylallyl caffeic acid has formula (C), pinobanksin-3-acetate has formula (D), tectochrysin has formula (E) pinostrobin chalcone has formula (F), benzyl ferulate has formula (G) and benzyl isoferulate has formula (H) shown below. Compounds B, C, G and H fit in formula (II) according to the invention.

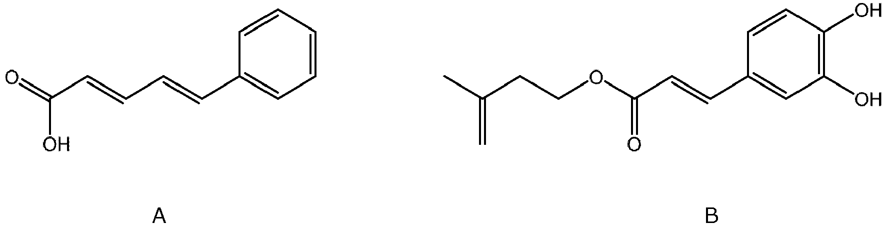

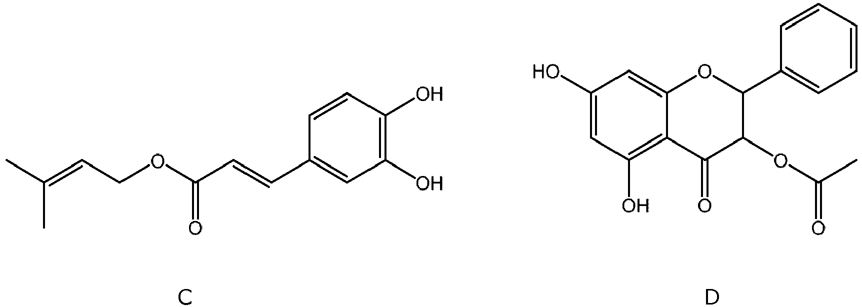

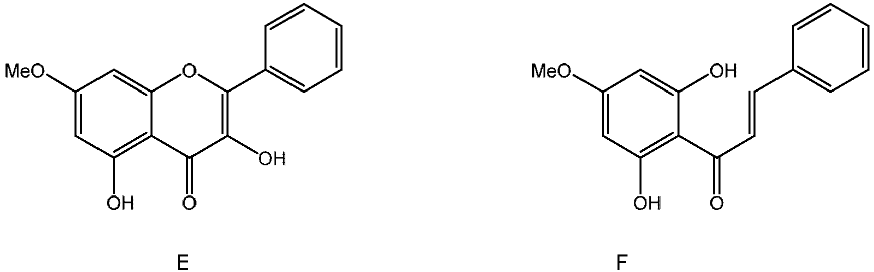

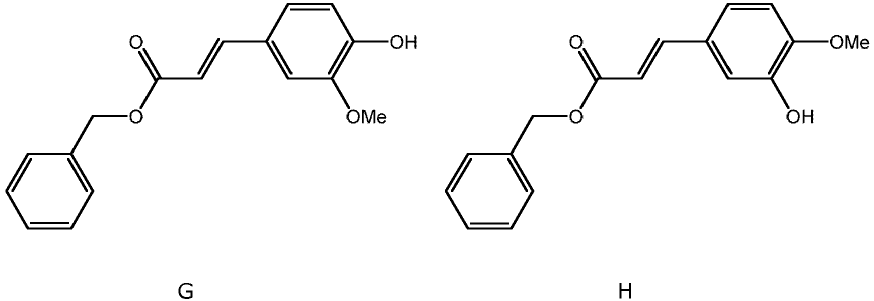

In one embodiment the anti-gastrointestinal cancer composition is an anti-colorectal cancer composition. In another embodiment the anti-gastrointestinal cancer composition is an anti-gastric cancer composition. In a further embodiment the anti-gastrointestinal cancer composition is an anti-throat cancer composition.

Described herein is a pharmaceutical composition comprising, consisting essentially or, or consisting of at least one compound as defined herein, including a compound of formula (I) or (II), or a compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone, ,
g) benzyl ferulate, and
h) benzyl isoferulate..

In one embodiment, the composition is for maintaining or improving gut health. Accordingly, described herein is a composition for improving gut health, the composition comprising, consisting essentially or, or consisting at least one compound as defined herein, including a compound of formula (I) or (II), or a compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate.

Described herein is a method of treating or preventing gastrointestinal cancer in a subject, the method comprising administering an effective amount of a composition comprising, consisting essentially or, or consisting of at least one compound as defined herein, including a compound of formula (I) or (II), or a compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate
to a subject in need thereof.

In one embodiment the gastrointestinal cancer is colorectal cancer. In another embodiment the gastrointestinal cancer is gastric cancer. In a further embodiment the gastrointestinal cancer is throat cancer.

Described herein is a method of inhibiting gastrointestinal tumour formation, inhibiting gastrointestinal tumour growth, inhibiting gastrointestinal tumour metastasis or treating or preventing gastrointestinal cancer in a subject, the method comprising separate, simultaneous or sequential administration of an effective amount of a composition comprising, consisting essentially or, or consisting of at least one compound as defined herein, including a compound of formula (I) or (II), or a compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate.
to a subject in need thereof.

In one embodiment the gastrointestinal tumour is a colorectal tumour. In another embodiment the gastrointestinal tumour is a gastric tumour. In a further embodiment the gastrointestinal tumour is a throat tumour.

Described herein is a method of inducing apoptosis of one or more neoplastic gastrointestinal cells in a subject, the method comprising administration of an effective amount of a composition comprising, consisting essentially or, or consisting of at least one compound as defined herein, including a compound of formula (I) or (II), or a compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate
to a subject in need thereof.

In one embodiment the apoptosis is of colorectal tumour cells. In another embodiment the apoptosis is of gastric tumour cells. In a further embodiment the apoptosis is of throat tumour cells.

Described herein is a method of modulating proliferation of one or more neoplastic gastrointestinal cells in a subject, the method comprising administration of an effective amount of a composition of the invention to a subject in need thereof.

For example in one embodiment the modulation is reduction. Accordingly described herein is a method of reducing proliferation of one or more neoplastic gastrointestinal cells in a subject, the method comprising administration of an effective amount of a composition comprising, consisting essentially of, or consisting of one or more compounds selected from 5-phenylpenta-2,4-dienoic acid, 3-methyl-3-butenyl caffeic acid, 1,1-dimethylallyl caffeic acid, pinobanksin-3-acetate, tectochrysin, pinostrobin chalcone, benzyl ferulate and benzyl isoferulate..

In one embodiment the proliferation is of colorectal tumour cells. In another embodiment the proliferation is of gastric tumour cells. In a further embodiment the proliferation is of throat tumour cells.

Described herein is a method of increasing the responsiveness of a subject to a gastrointestinal cancer therapy comprising administration to the subject of a composition of the invention as described herein.

In one embodiment the gastrointestinal cancer therapy is colorectal cancer therapy. In another embodiment the gastrointestinal cancer therapy is gastric cancer therapy. In a further embodiment the gastrointestinal cancer therapy is throat cancer therapy.

Described herein is a method of increasing the sensitivity of a gastrointestinal tumour in a subject to a cancer therapy comprising administration to the subject of a composition of the invention as described herein.

In one embodiment the gastrointestinal tumour is a colorectal tumour. In another embodiment the gastrointestinal tumour is a gastric tumour. In a further embodiment the gastrointestinal tumour is a throat tumour.

Described herein is a method of resensitising one or more gastrointestinal cancer cells that are resistant to treatment, the method comprising administering an effective amount of a composition of the invention as described herein to the one or more gastrointestinal cancer cells.

In one embodiment, the gastrointestinal cancer cells comprise a tumour present in a subject.

Described herein is a method of at least partially reversing the resistance of a neoplastic cell in a subject suffering from gastrointestinal cancer to a cancer therapy, the method comprising administration to the subject of a composition of the invention as described herein.

Described herein is a method of reversing, wholly or in part, the resistance of a gastrointestinal cancer-burdened patient to a gastrointestinal cancer therapy, the method comprising the step of administering to said patient a composition of the invention as described herein.

Described herein is a method of re-sensitising one or more tumours of a gastrointestinal cancer-burdened patient which are, or are predicted to either be or become, resistant to treatment with a gastrointestinal cancer therapy to treatment with a gastrointestinal cancer therapy, said method comprising the step of administering to said patient a composition of the invention as described herein.

In one embodiment, the tumours are resistant to treatment with a chemotherapeutic.

In one embodiment the gastrointestinal cancer is colorectal cancer. In another embodiment the gastrointestinal cancer is gastric cancer. In a further embodiment the gastrointestinal cancer is throat cancer.

Described herein is a method of improving gut health, the method comprising administering to a subject in need thereof a composition of the invention as described herein.

In one embodiment, the composition is a synergistic therapeutic composition. In one embodiment, the composition provides a synergistic therapeutic effect.

In one embodiment the composition comprises a compound described herein and at least one additional therapeutic agent that provide a synergistic therapeutic effect that is greater than the effect of either one alone or greater than the additive effects of either one alone. For example, there is a greater effect on induction of apoptosis, on gastrointestinal cancer cell survival or proliferation, on resensitisation to therapy, on treatment or prevention of gastrointestinal cancer, or the responsiveness of a subject or a tumour to the treatment method. In one embodiment, the compound and the at least one additional therapeutic agent allow the administration of a co-administered or sequentially administered gastrointestinal cancer therapy to be reduced or increased in dose or in length of administration, as appropriate.

In one embodiment the synergistic therapeutic composition comprises at least one additional compound or extract derived from propolis. For example, the composition additionally comprises at least one compound selected from the group comprising pinocembrin, CAPE, chrysin, galangin, benzyl caffeate or caffeic acid.

Described herein is a use of at least one compound as defined herein, including a compound of formula (I) or (II), or a compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate
in the manufacture of a composition for a purpose as herein described.

In one embodiment, the use is use together with at least one additional therapeutic agent in the manufacture of a composition for a purpose as herein described.

Described herein is a use of a composition comprising at least one compound as defined herein, including a compound of formula (I) or (II), or a compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate.
with at least one additional therapeutic agent in the manufacture of a composition for a purpose as herein described, wherein the composition is formulated to provide separate, simultaneous or sequential administration of the at least one compound and the at least one additional therapeutic agent.

In one embodiment the complex comprises cyclodextrin. In another embodiment the compound is provided dissolved in a solvent, for example ethanol or propylene glycol.

Described herein is a composition comprising, consisting essentially of or consisting of at least one compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate.

In one embodiment, the compound is isolated, purified or substantially purified.

Described herein is a composition to which has been added at least one compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate.

Described herein is a composition to which has been added at least one isolated, purified or substantially purified compound selected from any one or more of
a) 5-phenylpenta-2,4-dienoic acid,
b) 3-methyl-3-butenyl caffeic acid,
c) 1,1-dimethylallyl caffeic acid,
d) pinobanksin-3-acetate,
e) tectochrysin,
f) pinostrobin chalcone,
g) benzyl ferulate, and
h) benzyl isoferulate.

Described herein is a composition of the invention for use in inhibiting gastrointestinal tumour formation, inhibiting gastrointestinal tumour growth, inhibiting gastrointestinal tumour metastasis or treating or preventing gastrointestinal cancer in a human subject; inducing apoptosis of one or more neoplastic gastrointestinal cells in a human subject; increasing the responsiveness of a human subject to a gastrointestinal cancer therapy; increasing the sensitivity of a gastrointestinal tumour in a human subject to a gastrointestinal cancer therapy; resensitising one or more gastrointestinal cancer cells in a human subject that are resistant to treatment; at least partially reversing the resistance of a neoplastic cell in a human subject suffering from gastrointestinal cancer to a gastrointestinal cancer therapy; reversing, wholly or in part, the resistance of a gastrointestinal cancer-burdened human patient to a gastrointestinal cancer therapy; or re-sensitising one or more tumours of a gastrointestinal cancer-burdened human patient which are, or are predicted to either be or become, resistant to treatment with a gastrointestinal cancer therapy to treatment with a gastrointestinal cancer therapy

Described herein is a composition of the invention for use in the treatment or prevention of gastrointestinal cancer.

Described herein is a composition that has a 5-phenylpenta-2,4-dienoic acid concentration greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the composition has a 3-methyl-3-butenyl caffeic acid concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the composition has a 1,1-dimethylallyl caffeic acid concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a composition that has pinobanksin-3-acetate concentration greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a composition that has a tectochrysin concentration greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a composition that has a pinostrobin chalcone concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the composition has a benzyl ferulate concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the composition has a benzyl isoferulate concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the composition has a CAPE concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a composition that has a pinocembrin concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a composition that has a galangin concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a composition that has a chrysin concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a composition that has a pinobanksin concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a composition that has a caffeic acid concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the composition has a benzyl caffeate concentration of greater than about 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the one or more compounds are present in the anti-gastrointestinal cancer composition in the form of a propolis extract or a propolis fraction enriched in the one or more compounds selected from 3-methyl-3-butenyl caffeic acid, 1,1-dimethylallyl caffeic acid, benzyl ferulate and benzyl isoferulate. In one embodiment the enrichment is enrichment relative to the level present in crude propolis. In this embodiment the enrichment is enrichment relative to a non-lipid component present in dewaxed propolis. Thus, in one embodiment the enrichment contemplated is relative to a component present in the propolis extract or propolis fraction following dewaxing or other processing of crude propolis to remove wax, such as that present in crude or unprocessed propolis. In another embodiment, the enrichment is enrichment relative to one or more of the compounds identified in Table 1 herein.

In a further embodiment, the one or more compounds are present in the anti-gastrointestinal cancer composition in the form of a propolis extract or a propolis fraction enriched in the one or more compounds selected from 3-methyl-3-butenyl caffeic acid, 1,1-dimethylallyl caffeic acid benzyl ferulate and benzyl isoferulate, wherein the enrichment is relative to the level present in refined propolis. Thus, the enrichment contemplated is relative to a component present in the propolis extract or propolis fraction following dewaxing or other processing of crude propolis to remove wax, and then a further fractionation step or steps such as described in Example 1 and known in the art, for example separation into fractions using HPLC, column chromatography, adsorption and desorption from polymeric resins such as HP-20 or Sephadex, solvent partitioning.

In one embodiment the propolis extract or propolis fraction is enriched in 3-methyl-3-butenyl caffeic acid. In one embodiment the propolis extract or propolis fraction is enriched in 1,1-dimethylallyl caffeic acidIn one embodiment the propolis extract or propolis fraction is enriched in benzyl ferulate. In one embodiment the propolis extract or propolis fraction is enriched in benzyl isoferulate.

In various embodiments, the propolis extract or propolis fraction is enriched in any combination of two or more 3-methyl-3-butenyl caffeic acid, 1,1-dimethylallyl caffeic acidbenzyl ferulate and benzyl isoferulate.

Described herein is a propolis extract or propolis fraction that has a 5-phenylpenta-2,4-dienoic acid concentration greater than about 0.1, 0.5, 0.75, 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 0.1 to about 1, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the propolis extract or propolis fraction has a 3-methyl-3-butenyl caffeic acid concentration of greater than about 0.1, 0.5, 0.75, 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 0.1 to about 1, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the propolis extract or propolis fraction has a 1,1-dimethylallyl caffeic acid concentration of greater than about 0.1, 0.5, 0.75, 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 0.1 to about 1, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a the propolis extract or propolis fraction that has pinobanksin-3-acetate concentration of greater than about 0.1, 0.5, 0.75, 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 0.1 to about 1, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a propolis extract or propolis fraction that has a tectochrysin concentration of greater than about 0.1, 0.5, 0.75, 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 0.1 to about 1, about 1 to about 5, about 1 to about 10, about 2 to about 20, about 5 to about 20, about 5 to about 25, about 10 to about 25, about 10 to about 40, about 15 to about 100, or about 20 to about 999 mg/g).

Described herein is a propolis extract or propolis fraction that has a pinostrobin chalcone concentration of greater than about 0.1, 0.5, 0.75, 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 0.1 to about 1, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the propolis extract or propolis fraction has a benzyl ferulate concentration of greater than about 0.1, 0.5, 0.75, 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 0.1 to about 1, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the propolis extract or propolis fraction has a benzyl isoferulate concentration of greater than about 0.1, 0.5, 0.75, 1, 2, 3, 5, 10, 15, 20, 25, 30, 35, 40, 45, 50, 60, 70, 75, 80, 90, 100, 125, 150, 175, 200, 250, 300, 350, 400, 450, 500, 600, 700, 800, 900 or 999 mg/g and useful ranges may be selected between any of these values (for example, about 0.1 to about 1, about 1 to about 5, about 1 to about 10, about 2 to about 20, about 5 to about 20, about 5 to about 25, about 10 to about 25, about 10 to about 40, about 15 to about 100, or about 20 to about 999 mg/g).

In one embodiment, the propolis extract or propolis fraction is enriched in at least one of the compounds selected from the group comprising caffeic acid phenylether ester (CAPE), or benzyl caffeate.

In one embodiment, the propolis or propolis fraction has a CAPE concentration of greater than about 1 mg/g, than about 1.5 mg/g, than about 2 mg/g, about 2.5 mg/g, about 3 mg/g, about 3.5 mg/g, about 4 mg/g, about 4.5mg/g, about 5mg/g, about 5.5mg/g, about 6 mg/g, about 7.5 mg/g, about 10 mg/g, about 15 mg/g, about 20 mg/g, about 25 mg/g, about 30 mg/g, about 40 mg/g, about 50 mg/g, about 75 mg/g, about 100 mg/g, about 125 mg/g, about 150 mg/g, about 175 mg/g, about 200 mg/g, 250 mg/g, about 300 mg/g, about 350 mg/g, about 400 mg/g, about 450 mg/g, about 500 mg/g, about 550 mg/g, about 600 mg/g, about 650 mg/g, about 700 mg/g, about 750 mg/g, about 800 mg/g, about 850 mg/g, about 900 mg/g, about 950 mg/g, up to about 999 mg/g.

Described herein is a propolis or propolis fraction that has a pinocembrin concentration of greater than about 1 mg/g, than about 1.5 mg/g, than about 2 mg/g, about 2.5 mg/g, about 3 mg/g, about 3.5 mg/g, about 4 mg/g, about 4.5mg/g, about 5mg/g, about 5.5mg/g, about 6 mg/g, about 7.5 mg/g, about 10 mg/g, about 15 mg/g, about 20 mg/g, about 25 mg/g, about 30 mg/g, about 40 mg/g, about 50 mg/g, about 75 mg/g, about 100 mg/g, about 125 mg/g, about 150 mg/g, about 175 mg/g, about 200 mg/g, 250 mg/g, about 300 mg/g, about 350 mg/g, about 400 mg/g, about 450 mg/g, about 500 mg/g, about 550 mg/g, about 600 mg/g, about 650 mg/g, about 700 mg/g, about 750 mg/g, about 800 mg/g, about 850 mg/g, about 900 mg/g, about 950 mg/g, up to about 999 mg/g.

Described herein is a propolis or propolis fraction that has a galangin concentration of greater than about 1 mg/g, than about 1.5 mg/g, than about 2 mg/g, about 2.5 mg/g, about 3 mg/g, about 3.5 mg/g, about 4 mg/g, about 4.5mg/g, about 5mg/g, about 5.5mg/g, about 6 mg/g, about 7.5 mg/g, about 10 mg/g, about 15 mg/g, about 20 mg/g, about 25 mg/g, about 30 mg/g, about 40 mg/g, about 50 mg/g, about 75 mg/g, about 100 mg/g, about 125 mg/g, about 150 mg/g, about 175 mg/g, about 200 mg/g, 250 mg/g, about 300 mg/g, about 350 mg/g, about 400 mg/g, about 450 mg/g, about 500 mg/g, about 550 mg/g, about 600 mg/g, about 650 mg/g, about 700 mg/g, about 750 mg/g, about 800 mg/g, about 850 mg/g, about 900 mg/g, about 950 mg/g, up to about 999 mg/g.

Described herein is a propolis or propolis fraction that has chrysin concentration of greater than about 1 mg/g, than about 1.5 mg/g, than about 2 mg/g, about 2.5 mg/g, about 3 mg/g, about 3.5 mg/g, about 4 mg/g, about 4.5mg/g, about 5mg/g, about 5.5mg/g, about 6 mg/g, about 7.5 mg/g, about 10 mg/g, about 15 mg/g, about 20 mg/g, about 25 mg/g, about 30 mg/g, about 40 mg/g, about 50 mg/g, about 75 mg/g, about 100 mg/g, about 125 mg/g, about 150 mg/g, about 175 mg/g, about 200 mg/g, 250 mg/g, about 300 mg/g, about 350 mg/g, about 400 mg/g, about 450 mg/g, about 500 mg/g, about 550 mg/g, about 600 mg/g, about 650 mg/g, about 700 mg/g, about 750 mg/g, about 800 mg/g, about 850 mg/g, about 900 mg/g, about 950 mg/g, up to about 999 mg/g.

In one embodiment, the propolis or propolis fraction has benzyl caffeate concentration of greater than about 1 mg/g, than about 1.5 mg/g, than about 2 mg/g, about 2.5 mg/g, about 3 mg/g, about 3.5 mg/g, about 4 mg/g, about 4.5mg/g, about 5mg/g, about 5.5mg/g, about 6 mg/g, about 7.5 mg/g, about 10 mg/g, about 15 mg/g, about 20 mg/g, about 25 mg/g, about 30 mg/g, about 40 mg/g, about 50 mg/g, about 75 mg/g, about 100 mg/g, about 125 mg/g, about 150 mg/g, about 175 mg/g, about 200 mg/g, 250 mg/g, about 300 mg/g, about 350 mg/g, about 400 mg/g, about 450 mg/g, about 500 mg/g, about 550 mg/g, about 600 mg/g, about 650 mg/g, about 700 mg/g, about 750 mg/g, about 800 mg/g, about 850 mg/g, about 900 mg/g, about 950 mg/g, up to about 999 mg/g.

Described herein is a propolis or propolis fraction that has a caffeic acid concentration of greater than about 1 mg/g, than about 1.5 mg/g, than about 2 mg/g, about 2.5 mg/g, about 3 mg/g, about 3.5 mg/g, about 4 mg/g, about 4.5mg/g, about 5mg/g, about 5.5mg/g, about 6 mg/g, about 7.5 mg/g, about 10 mg/g, about 15 mg/g, about 20 mg/g, about 25 mg/g, about 30 mg/g, about 40 mg/g, about 50 mg/g, about 75 mg/g, about 100 mg/g, about 125 mg/g, about 150 mg/g, about 175 mg/g, about 200 mg/g, 250 mg/g, about 300 mg/g, about 350 mg/g, about 400 mg/g, about 450 mg/g, about 500 mg/g, about 550 mg/g, about 600 mg/g, about 650 mg/g, about 700 mg/g, about 750 mg/g, about 800 mg/g, about 850 mg/g, about 900 mg/g, about 950 mg/g, up to about 999 mg/g.

In various embodiments, the composition comprises or is administered separately, simultaneously or sequentially with at least one additional therapeutic agent, preferably the at least one additional therapeutic agent is an anti-tumour agent, preferably the anti-tumour agent is selected from an anti-tumour food factor, a chemotherapeutic agent, or an immunotherapeutic agent.

In various embodiments, the gastrointestinal cancer therapy, the therapeutic agent, or the anti-tumour agent is effective to induce apoptosis, for example, induce apoptosis in one or more gastrointestinal cancer cells or in one or more neoplastic cells.

In one embodiment, the gastrointestinal cancer therapy, the therapeutic agent, or the anti-tumour agent is butyrate or a source of butyrate.

In one embodiment, the composition is a consumer good.

In one embodiment the composition is a food, drink, food additive, drink additive, dietary supplement, nutritional product, medical food, nutraceutical, medicament or pharmaceutical.

In various embodiments, the composition may be formulated for oral, topical, or parenteral administration.

In one embodiment, the composition comprises one or more additional anti-gastrointestinal cancer agents.

In one embodiment, the composition is a pharmaceutical composition. In another embodiment, the composition is a nutraceutical composition.

In various embodiments, the chemotherapeutic agent is selected from the group comprising mitotic inhibitors, such as vinca alkaloids, including vincristine, vinblastine, vinorelbine, vindesine, vinflunine, podophyllotoxin, taxanes, including docetaxel, larotaxel, ortataxel, paclitaxel, and tesetaxel, and epothilones, such as ixabepilone; topoisomerase I inhibitors, such as topotecan, irinotecan, camptothecin, rubitecan, and belotecan, topoisomerase type II inhibitors, including amsacrine, etoposide, etoposide phosphate, and teniposide, anthracyclines, such as aclarubicin, daunorubicin, doxorubicin, epirubicin, idarubicin, amrubicin, pirarubicin, valrubicin, and zorubicin, and anthracenediones, such mitoxantrone and pixantrone; antimetabolites, including dihydrofolate reductase inhibitors, such as aminopterin, methotrexate, pemetrexed, thymidylate synthase inhibitors, such as raltitrexed and pemetrexed, adenosine deaminase inhibitors, including pentostatin, halogenated or ribonucleotide reductase inhibitors, such as cladribine, clofarabine, and fludarabine, thiopurines, including thioguanine and mercaptopurine, thymidylate synthase inhibitors, including fluorouracil, capecitabine, tegafur, carmofur, and floxuridine, DNA polymerase inhibitors, such as cytarabine, ribonucleotide reductase inhibitors, such as gemcitabine, hypomethylating agents, including azacitidine, and decitabine, and ribonucleotide reductase inhibitors, such as hydroxyurea; cell-cycle nonspecific antineoplastic agents, including alkylating agents such as nitrogen mustards, including mechlorethamine, cyclophosphamide, ifosfamide, trofosfamide, chlorambucil, melphalan, prednimustine, bendamustine, uramustine, estramustine, nitrosoureas, including carmustine, lomustine, semustine, fotemustine, nimustine, ranimustine, and streptozocin, alkyl sulfonates, including busulfan, mannosulfan, and treosulfan, aziridines, including carboquone, thioTEPA, triaziquone, and triethylenemelamine, alkylating-like agents, including platinum agents such as cisplatin, carboplatin, oxaliplatin, nedaplatin, triplatin tetranitrate, satraplatin, hydrazines, such as procarbazine, triazenes, such as dacarbazine, temozolomide, altretamine, and mitobronitol, and streptomycins, such as actinomycin, bleomycin, daunomycin, mitomycin, and plicamycin; photosensitizers, including aminolevulinic acid, methyl aminolevulinate, efaproxiral, and porphyrin derivatives, such as porfimer sodium, talaporfin, temoporfin, and verteporfin; enzyme inhibitors, including farnesyltransferase inhibitors such as tipifarnib, cyclin-dependent kinase inhibitors, such as alvocidib and seliciclib, proteasome inhibitors, such as bortezomib, phosphodiesterase inhibitors, such as anagrelide, IMP dehydrogenase inhibitors, such as tiazofurine, lipoxygenase inhibitors, such as masoprocol, and PARP inhibitors, such as olaparib; receptor antagonists, such as endothelin receptor antagonists including atrasentan, retinoid X receptor antagonists, such as bexarotene, and testolactone; and other chemotherapeutics, including amsacrine, trabectedin, retinoids such as alitretinoin and tretinoin, arsenic trioxide, asparagine depleters such as asparaginase or pegaspargase, celecoxib, demecolcine, elesclomol, elsamitrucin, etoglucid, and lonidamine.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

In this specification where reference has been made to patent specifications, other external documents, or other sources of information, this is generally for the purpose of providing a context for discussing the features of the invention.

### DETAILED DESCRIPTION

The present invention is based on the finding that compositions comprising compounds selected from any one or more of 3-methyl-3-butenyl caffeic acid, 1,1-dimethylallyl caffeic acid, benzyl ferulate and benzyl isoferulate enhance the activity and physicochemical properties of propolis or materials with propolis contained.

Accordingly, provided that the anti-gastrointestinal cancer compositions are formulated so as to be suitable for administration to a mammalian subject, for example they consist of materials that are safe to the human body, they can be used for manufacturing anti-gastrointestinal cancer pharmaceutical compositions and drugs, as well as nutraceutical compositions, consumer goods, such as beverages, foods, and the like.

Furthermore, as the anti-gastrointestinal cancer activity of embodiments of the compositions of the invention is maintained for a sustained period, the dosage or frequency of administration of the composition can be reduced, or higher activity is provided, or both.

The phrases "anti-gastrointestinal cancer compositions" or "compositions having anti-gastrointestinal cancer activity" (used interchangeably herein) contemplate any kind of composition suitable for administration to a subject. Examples include anti-colorectal cancer, anti-gastric cancer or anti-throat cancer compositions containing compounds derived from propolis.

The term "and/or" can mean "and" or "or".

The terms "cancer" and "cancerous" refer to a physiological condition in mammals that is typically characterized by abnormal or unregulated cell proliferation, cell survival, cell motility, neoplasticity, and/or oncogenicity. Cancer and cancer pathology can be associated, for example, with metastasis, interference with the normal functioning of neighbouring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc. Specifically included are colorectal cancers and precancerous conditions, which can include epithelial tumours, nonepithelial tumours, carcinomas, for example, carcinomas in situ, as well as invasive colorectal cancers. Also included are gastric cancers and precancerous conditions, which can include epithelial tumours, adenocarcinomas, gastric lymphomas, carcinoid tumours, stromal tumours. Also included are throat cancers and precancerous conditions, which can include epithelial tumours, squamous cell carcinomas, adenocarcinomas. Cancers may be, for example, carcinomas in situ, as well as invasive cancers.

According to the invention, the gastrointestinal cancer is gastric cancer.

The term "comprising" as used in this specification means "consisting at least in part of". When interpreting statements in this specification that include that term, the features, prefaced by that term in each statement, all need to be present but other features can also be present. Related terms such as "comprise" and "comprised" are to be interpreted in the same manner.

An "effective amount" is the amount required to confer therapeutic effect. The interrelationship of dosages for animals and humans (based on milligrams per meter squared of body surface) is described by Freireich, et al. (1966). Body surface area can be approximately determined from height and weight of the subject. See, e.g., Scientific Tables, Geigy Pharmaceuticals, Ardley, New York, 1970, 537. Effective doses also vary, as recognized by those skilled in the art, dependent on route of administration, excipient usage, and the like.

As used herein, an "extract" or a "fraction" of propolis suitable for use in the present invention is enriched in one or more of the compounds described herein such that they exhibit anti-gastrointestinal cancer activity. Such functional extracts or functional fractions may have greater or lesser activity than native propolis. In one example, one or more of the biological activities of the native propolis possessed by the functional extract or functional fraction may be present to a greater or lesser degree in the functional extract or functional fraction than is found in the native propolis. In another example, each of the biological activities of the native propolis possessed by the functional extract or functional fraction is present to a greater or lesser degree in the functional extract or functional fraction than is found in the native propolis. In still a further example, it may be desirable to provide a functional extract or functional fraction in which one or more of the biological activities of the native propolis is maintained or is present to a greater degree than is found in the native propolis, but one or more other biological activities of the native propolis is not present or is present to a lesser degree than is found in the native propolis. Examples of such functional extracts include the anti-gastrointestinal cancer tincture described herein in the Examples.

Methods and assays to determine one or more biological effects elicited by the compounds described herein are well known in the art and examples are described herein, and such methods and assays can be used to identify or verify one or more functional extracts or functional fractions of propolis. For example, an assay of the ability of propolis to increase one or more oncogenic traits in a cell, such as those described herein in the Examples, is amenable to identifying one or more functional extracts or functional fractions of propolis.

As used herein, "propolis" contemplates propolis produced by bees from any botantical source capable of providing one or more of the compounds discussed herein. In one embodiment, the propolis is "European" propolis. "European" propolis is also known under different names, such as "Poplar" propolis. For example, the propolis is derived from the bud and leaf exudates of one or more species of poplars, birches, larches or willows. Propolis has been classified into seven major classes based on plant source (Sforcin and Bankova, 2011. Propolis: is there a potential for the development of new drugs? J. Ethnopharmacology, 133: 253-260.). These classes are "Poplar" from Europe, North America, Southern South America, New Zealand; "Brazilian green", which contains artepillan C; "Birch" from Russia; "Red propolis" from Cuba, Brazil, Mexico; "Mediterranean" from Greece, Sicily, Crete, Malta and sourced from conifers; "Clusia" from Cuba and Venezuela; and "Pacific" from Okinawa, Taiwan, Indonesia, which contain 'propolins'.

Exemplary compounds and concentrations of the compounds reported in propolis from various countries are presented in Table 1 below. These compounds are useful, for example in identifying the source of propolis, or in characterizing and identifying propolis suitable for use in the present disclosure.

**Table 1: Compositional data for ethanol soluble extract of propolis from various geographic regions (from Kumazawa et al., 2004. Antioxidant activity of propolis of various geographic regions, Food Chemistry 84: 329-339)**

| | Arg | Aus | Bra | Bul | Chil | Chi a | Chi b | Chi c | Hun | SA | Ukr | Uru | USA | Uzb | NZ | MHNZ1 | MHNZ2 |
|---|---|---|---|---|---|---|---|---|---|---|---|---|---|---|---|---|---|
| Caffeic acid | 0.7 | 1.7 | 1.6 | 7.2 | 0.4 | 3.3 | 2.8 | 2.4 | 3.1 | 0.2 | 0.8 | 0.7 | 0.8 | 1.5 | 2.8 | 9.4 | 7.2 |
| *p*-Coumaric | 1.8 | 3.6 | 27.4 | 3.5 | 1.9 | 4 | 4 | 3 | 3.7 | 1.5 | 8.9 | 8.4 | 19.4 | 0.9 | 3.1 | 3.4 | 2.6 |
| 3,4-Diemethoxy cinnamic acid | 2.2 | 8.6 | | 4 | 1.8 | 10.1 | 7.4 | 7.9 | 5.2 | | 0.8 | 1.1 | 2 | 2.6 | 9.2 | | |
| Quercetin | 2.2 | 4.8 | | 4.7 | 1.5 | 4.4 | 3.8 | .8 | 4.4 | | | 2.5 | 3.8 | 0.8 | 1.2 | | |
| Pinobanksin-5-methyl ether | 15 | 23.8 | | 19.7 | 18.8 | 19.8 | 26.2 | 21.1 | 21.8 | 5.9 | 7.5 | 51 | 23.8 | 10.8 | 20 | | |
| Apigenin | 12 | 18.4 | | 13.4 | 14.2 | 17.1 | 17.1 | 14.3 | 9 | | 3.9 | 14.8 | 0.6 | 8.1 | 78.3 | | |
| Kaemferol | 2.3 | 3.9 | | 5 | 1.4 | 2.1 | 2.6 | 2.5 | 4.8 | 1 | 10.9 | 2.5 | 10.3 | 3.8 | 3.7 | | |
| Pinobanksin | 22. 5 | 32.1 | | 84.8 | 21.4 | 36.1 | 35 | 22.5 | 21.3 | 31.4 | 6.6 | 36.5 | 23.2 | 29.4 | 36.3 | 35.2 | 25 |
| Cinnamylidene-a ¹ | 30. 4 | 14.6 | | 6.3 | 31.2 | 11.2 | 12.7 | 10.5 | 7.8 | | | 13.7 | 5.2 | 2.9 | 18.1 | | |
| Chrysin | 68. 5 | 139 | | 120 | 66.3 | 127. 3 | 138 | 138 | 82.9 | 11.2 | 12.9 | 77.3 | 39.4 | 87 | 102 | 32.9 | 24.9 |
| Pinocembrin | 68. 7 | 58.7 | | 94.4 | 86.2 | 54.8 | 61.5 | 46.9 | 51.2 | 69.8 | 9.2 | 75 | 46.7 | 44.5 | 100 | 119 | 95.7 |
| Galangin | 32. 5 | 42.5 | | 45.6 | 37.7 | 39.6 | 33.5 | 32.6 | 44.2 | 18.9 | 13.4 | 48.8 | 21.5 | 41.4 | 58.2 | 50.6 | 37.3 |
| Pinobanksin-3-acetate | 56. 3 | 79.7 | | 41.2 | 63.4 | 52.5 | 64.2 | 51.2 | 59.9 | 7.7 | 14.7 | 80 | 27.6 | 58.4 | 66.2 | 71.3 | 65.5 |
| CAPE | 8.6 | 10.4 | | 5.6 | 7.4 | 29.2 | 24.5 | 19.3 | 15.4 | | 2.6 | 12.4 | 7.2 | 18.6 | 12 | 9.4 | 6.1 |
| Cinnamyl caffeate | 6.6 | 16.6 | | 0.7 | 6.1 | 16.3 | 20.3 | 14.4 | 13.8 | | 2.1 | 8.9 | 7.5 | 2.2 | 12.7 | | |
| Tectochrysin | 31. 4 | 58.2 | | 96.9 | 33.1 | 62 | 45.4 | 35.5 | 39.0 | 7.7 | 12.4 | 23.8 | 36.1 | 7.3 | 62.2 | 3.5 | 3.3 |
| Artepillin-c | | | 43.9 | | | | | | | | | | | | | | |

| | | | | | | | | | | | | | | | | | |
|---|---|---|---|---|---|---|---|---|---|---|---|---|---|---|---|---|---|
| All values are mg/g propolis resin. ¹Cinnamylideneacetic acid (or phenyl petadienoic acid). Arg = Argentina, Aus = Australia, Bra = Brazilian green? propolis, Bul = Bulgaria, Chil = Chile, Chi = Chinese (a = Hebei, b = Hubei and c = Zheijiang province), Hun = Hungary, NZ = New Zealand, SA = South Africa, Ukr = Ukraine, Uru = Uruguay, Uzb = Uzbekistan, MHNZ 1 = New Zealand propolis (Manuka Health NZ Ltd), MHNZ 2 = New Zealand propolis (Manuka Health NZ Ltd). | | | | | | | | | | | | | | | | | |

When used in respect of an agent having anti-gastrointestinal cancer activity, such as a composition of the disclosure or a component of a composition of the disclosure, the phrase "retaining anti-gastrointestinal cancer activity" and grammatical equivalents and derivatives thereof is intended to mean that the agent still has useful anti-gastrointestinal cancer activity. Preferably, the retained activity is at least about 35, 40, 45, 50, 55, 60, 65, 70, 75, 80, 85, 90, 95, 99 or 100% of the original activity, and useful ranges may be selected between any of these values (for example, from about 35 to about 100%, from about 50 to about 100%, from about 60 to about 100%, from about 70 to about 100%, from about 80 to about 100%, and from about 90 to about 100%). Exemplary compositions of the disclosure are capable of supporting the maintenance of useful anti-gastrointestinal cancer activity of the anti-gastrointestinal cancer agent (s) they comprise, and can be said to retain anti-gastrointestinal cancer activity, ideally until utilized in the methods contemplated herein.

When used in respect of a composition of the disclosure or a component of a composition of the disclosure, the phrase "enhancing anti-gastrointestinal cancer activity" and grammatical equivalents and derivatives thereof is intended to mean that when present in the composition, an equivalent amount or concentration of the anti-gastrointestinal cancer agent has increased anti-gastrointestinal cancer activity compared to that of the agent in the absence of the composition (such as the isolated agent). Preferably, the enhanced activity is at least about 105, 110, 115, 120, 125, 130, 135, 140, 145, 150, 155, 160, 165, 170, 175, 180, 185, 190, 195, 200%, or more of the original activity, and useful ranges may be selected between any of these values (for example, from about 105 to about 150%, from about 120 to about 180%, from about 140 to about 200%, from about 160 to about 200%, from about 180 to about 200%, and from about 190 to about 200%). In certain embodiments, compositions of the disclosure may exhibit enhanced anti-gastrointestinal cancer activity, that is, exhibit at least about 105, 110, 115, 120, 125, 130, 135, 140, 145, 150, 155, 160, 165, 170, 175, 180, 185, 190, 195, 200%, or more of the anti-gastrointestinal cancer activity of propolis alone. Similarly, preferred compositions of the disclosure are capable of supporting the maintenance of enhanced anti-gastrointestinal cancer activity, and can be said to retain enhanced anti-gastrointestinal cancer activity, ideally until utilised using the methods contemplated herein. The enhanced activity (including enhanced maintenance of activity) is believed, without wishing to be bound by any theory, to result from synergy amongst the various components of the compositions of the disclosure.

As used herein, the term "stable" when used in relation to a composition of the disclosure means a composition capable of supporting anti-gastrointestinal cancer activity for preferably more than two hours, more than three hours, 6 hours, 9 hours, 12 hours, 15 hours, 18 hours, 20 hours, more than one day, preferably about two, about three, about four, preferably about five, more preferably about six days, preferably a week, two weeks, three weeks, a month, or longer.

The term "oral administration" includes oral, buccal, enteral and intra-gastric administration.

The term "parenteral administration" includes but is not limited to topical (including administration to any dermal, epidermal or mucosal surface), subcutaneous, intravenous, intraperitoneal, intramuscular and intratumoural (including any direct administration to a tumour) administration.

The term "pharmaceutically acceptable carrier" is intended to refer to a carrier including but not limited to an excipient, diluent or auxiliary that can be administered to a subject as a component of a composition of the invention. Preferred carriers do not reduce the activity of the composition and are not toxic when administered in doses sufficient to deliver an effective amount of any one or more of 3-methyl-3-butenyl caffeic acid, 1,1-dimethylallyl caffeic acid benzyl ferulate or benzyl isoferulate, or, when administered, of another anti-gastrointestinal cancer agent.

The term "(s)" following a noun contemplates the singular or plural form, or both.

The term "subject" is intended to refer to an animal, preferably a mammal, more preferably a mammalian companion animal or human. Preferred companion animals include cats, dogs and horses. Other mammalian subjects include an agricultural animal, including a horse, a pig, a sheep, a goat, a cow, a deer, or a fowl, or a laboratory animal, including a monkey, a rat, or a mouse.

The term "treat" and its derivatives should be interpreted in their broadest possible context. The term should not be taken to imply that a subject is treated until total recovery. Accordingly, "treat" broadly includes maintaining a subject's disease progression or symptoms at a substantially static level, increasing a subject's rate of recovery, amelioration and/or prevention of the onset of the symptoms or severity of a particular condition, or extending a patient's quality of life. The term "treat" also broadly includes the maintenance of good health for sensitive individuals and building stamina for disease prevention.

### Exemplary uses of the disclosure

The methods and compositions of the disclosure may be used in the treatment or prevention of colorectal cancers, neoplastic disorders associated with colorectal cancer cells, and the symptoms of colorectal cancer, colorectal cancer treatment, and associated disorders.

Colorectal cancer is a neoplastic condition affecting the large intestine, particularly the colon and rectum - hence it is commonly referred to as colon cancer or bowel cancer. Risk factors include age, diet, in particular high intake of fat, alcohol or red meat, male gender, obesity, smoking and lack of exercise.

Colorectal cancer originates from epithelial cells in the colon or rectum of the gastrointestinal tract, and is most commonly associated with defects in the Wnt-APC-beta-catenin signaling pathway. Where possible, the preferred treatment is complete surgical removal which can be curative. If metastasis has occurred and the cancer has entered the lymph nodes, the chemotherapeutic agents fluorouracil or capecitabine have been reported to increase life expectancy. Other chemotherapeutics considered for use include fluorouracil, capecitabine, UFT, leucovorin, irinotecan, or oxaliplatin, and combinations of these agents. However, where the cancer is more widespread, or when surgery is not possible, treatment generally focuses on palliative care and extension of life expectancy. Combinations of radiation and chemotherapy can be considered for rectal cancer, but radiotherapy is not typically used in colon cancer due to radiosensitivity of the bowels.

The methods and compositions of the disclosure may be used in the treatment or prevention of gastric cancers, neoplastic disorders associated with gastric cancer cells, and the symptoms of gastric cancer, gastric cancer treatment, and associated disorders. According to the invention, the compounds and compositions are used for the treatment or prevention of a gastric tumour or gastric cancer.

Gastric cancer is a neoplastic condition arising from any part of the stomach. Prognosis is poor because most patients present with advanced disease. Risk factors include age, diet, in particular high intake of smoked foods, salted fish, cured meats and pickled vegetables, smoking, male gender and a history of autoimmune atrophic gastritis or intestinal metaplasia.

Approximately 90% of cancers are adenocarcinomas, originating in the glandular epithelium of the gastric mucosa. Other types include lymphomas (MALTomas OR MALT lymphoma), and carcinoid and stromal tumours. Where possible, the preferred treatment is complete surgical removal of all or part of the stomach and surrounding lymph nodes. Gastric cancers are not particularly sensitive to chemotherapeutic agents, however, chemotherapeutics including fluorouracil, capecitabine, BCNU (carmustine), methyl-CCNU (Semustine), and doxorubicin (Adriamycin), Mitomycin C, cisplatin and taxotere have been used to in palliative care regimes. Combinations of radiation and chemotherapy are sometimes used for the treatment of gastric cancers.

The methods and compositions of the disclosure may be used in the treatment or prevention of throat cancers, neoplastic disorders associated with throat cancer cells, and the symptoms of throat cancer, throat cancer treatment, and associated disorders.

Throat cancer, also referred to as oesophaegeal cancer, pharyngeal cancer, or laryngeal cancer, encompasses tumours that develop in the tissues of the pharynx, nasopharynx, oropharynx, hypopharynx, larynx (voice box) or tonsils. Approximately 90% of throat cancers are squamous cell carcinomas originating from the mucosal lining (epithelium) of these regions. Risk factors include diet, in particular high intake of alcohol, smoking, use of smokeless tobacco, betel nut chewing and exposure to environmental carcinogens including occupational exposures nickel refining, textile fibres and woodworking.

The most common therapies are surgical excision of tumours and radiation therapy. Chemotherapeutic agents may be used in combination surgery and/or radiation. Agents include paclitaxel, carboplatin, cetuximab, taxotere and docetaxel.

Robust gut health is associated with intestinal comfort, resistance to infectious diseases and the prevention of chronic gastrointestinal diseases.

This includes the treatment or prevention of a condition associated with poor gut health, low immunity and gastrointestinal tract inflammation. For example, the methods described herein and compositions of the disclosure are useful for the treatment or prevention of inflammatory bowel disease, irritable bowel syndrome, environmental enteropathy, infectious diarrhea and for the removal or alleviation of visceral pain.

### Compositions for use in the invention

Exemplary anti-gastrointestinal cancer compositions described herein include pharmaceutical compositions. Exemplary compositions described herein for use in the maintenance of gut health include pharmaceutical compositions and nutraceutical compositions, along with consumer products and the like.

Other anti-gastrointestinal cancer substances generally known can be combined with the anti-gastrointestinal cancer compositions described herein, depending upon the application to which the composition is to be put.

Compositions suitable for administration to a subject may be formulated as a food, drink, food additive, drink additive, dietary supplement, nutritional product, medical food, nutraceutical, medical supply, medical device, medicament or pharmaceutical. Appropriate formulations may be prepared by an art skilled worker with regard to that skill and the teaching of this specification.

Described herein is a use of one or more of the compounds described herein, optionally with at least one anti-gastrointestinal cancer agent, or of one or more propolis fractions or propolis extracts enriched in one or more of the compounds described herein, in the manufacture of a food, drink, food additive, drink additive, dietary supplement, nutritional product, medical food, nutraceutical, medical device, medical supply, medicament or pharmaceutical. In one embodiment, the composition is formulated for oral administration. In another embodiement, the composition is formulated for parenteral, including topical, administration. In certain embodiments, the composition is for inducing apoptosis, treating or preventing gastrointestinal cancer, or one or more other uses as described above.

In one embodiment the composition is in the form of a powder, a tablet, a caplet, a pill, a hard or soft capsule or a lozenge.

In one embodiment the composition is in the form of a sachet, a dispensable powder, granules, a suspension, an elixir, a liquid, a drink, or any other form that can be added to food or drink, including for example water or fruit juice. In one embodiment the composition is an enteral product, a solid enteral product or a liquid enteral product.

In one embodiment the composition further comprises one or more constituents (such as antioxidants) which prevent or reduce degradation of the composition during storage or after administration.

In one embodiment, compositions useful herein include any edible consumer product which is able to carry one or more cyclodextrins. When the composition comprises as the at least one additional anti-gastrointestinal cancer agent as a proteinaceous factor, the edible consumer product is one able to carry protein.

In various embodiments, the composition comprises cyclodextrin. In one embodiment, the cyclodextrin is gamma-cyclodextrin, or the cyclodextrin is present as a combination of cyclodextrins comprising gamma-cyclodextrin.

In one embodiment, the cyclodextrin is chemically-modified cyclodextrin.

Examples of suitable edible consumer products include baked goods, powders, liquids, confectionary products, reconstituted fruit products, snack bars, food bards muesli bars, spreads, sauces, dips, dairy products including ice creams, yoghurts and cheeses, drinks including dairy and non-dairy based drinks (such as milk drinks including milk shakes, and yogurt drinks), milk powders, sports or nutritional supplements including dairy and non-dairy based sports or nutritional supplements, food additives such as protein sprinkles and dietary supplement products including daily supplement tablets. Within this embodiment, a composition useful herein may also be an infant formula, in powder or liquid form. Suitable nutraceutical compositions useful herein may be provided in similar forms. Particularly contemplated are compositions additionally comprising milk or one or more milk products or components of milk, such as milk protein, whey protein, colostrums, milk fat, or any fractions of milk or one or more milk products or components of milk, such as a milk fat fraction, a milk protein fraction, a whey protein fraction, a colostrums fraction, or the like.

Compositions useful herein may further include other factors such as calcium, zinc, magnesium, selenium, vitamin C, vitamin D, vitamin E, vitamin K2, complex carbohydrates, edible or cooking oils including palm, olive, soybean, canola, corn, sunflower, safflower, peanut, grape seed, sesame, nut, almond, cashew, hazelnut, macadamia, pecan, pistachio, and walnut, and other edibles include acai, amaranth, apricot, argan, artichoke, avocado, babassu, ben, blackcurrant seed, borage seed, borneo tallow nut, bottle gourd, buffalo gourd, carob pod (algaroba), cohune, coriander seed, evening primrose, false flax, hemp, kapok seed, lallemantia, meadowfoam seed, mustard, okra seed (hibiscus seed), perilla seed, pequi, pine nut, poppyseed, prune kernel, pumpkin seed, quinoa, ramtil, rice bran, tea (camellia), thistle, watermelon seed, or wheat germ oil, or a combination thereof.

The compositions useful herein may be formulated to allow for administration to a subject by any chosen route, including but not limited to oral or parenteral (including topical, subcutaneous, intramuscular and intravenous) administration. Those skilled in the art will appreciate that the route of administration to a subject will typically take into account the purpose for which the composition is being administered - for example, where a pharmaceutical composition of the invention is being administered to maintain gut health, the route of administration will typically be chosen taking into account the nature of this disorder.

In general, for oral administration a dietary (a food, food additive or food supplement for example), nutraceutical or pharmaceutical composition useful herein may be formulated by a skilled worker according to known formulation techniques.

Thus, a pharmaceutical composition useful according to the invention may be formulated with an appropriate pharmaceutically acceptable carrier (including excipients, diluents, auxiliaries, and combinations thereof) selected with regard to the intended route of administration and standard pharmaceutical practice. See for example, Remington's Pharmaceutical Sciences, 16th edition, Osol, A. Ed., Mack Publishing Co., 1980.

While the preferred route of administration is oral, it should be understood that any mode of administration may be suitable for any composition of the invention, including administration by multiple routes, including different routes for different agents. Therefore, inhalation (nasal or buccal inhalation) and vaginal and rectal administration of any composition of the invention is also contemplated. Intramedullar, epidural, intra-articular, and intra-pleural administration of any composition of the invention is also contemplated. Administration of a composition of the invention, optionally with at least one additional anti-gastrointestinal cancer factor, by a first administration route accompanied by separate, simultaneous or sequential administration of one or more other agents, including one or more other anti-gastrointestinal cancer agents, by a second administration route is also contemplated; for example, oral administration of a composition of the invention accompanied by topical administration of the at least one additional anti-gastrointestinal cancer agent.

The compositions of the invention may also be formulated as a dosage form. A dosage form useful herein may be administered orally as a powder, liquid, tablet or capsule. Suitable dosage forms may contain additional agents as required, including emulsifying, antioxidant, flavouring or colouring agents, or have an enteric coating. Suitable enteric coatings are known. Enteric coatings surrounding the active ingredients and prevent the release of the active ingredients in the stomach but allow release after the dosage form has left the stomach. Dosage forms useful herein may be adapted for immediate, delayed, modified, sustained, pulsed or controlled release of the active components. Suitable formulations may contain additional agents as required, including emulsifying, antioxidant, flavouring or colouring agents.

Capsules can contain any standard pharmaceutically acceptable materials such as gelatin or cellulose. Tablets can be formulated in accordance with conventional procedures by compressing mixtures of the active ingredients with a solid carrier and a lubricant. Examples of solid carriers include starch and sugar bentonite. Active ingredients can also be administered in a form of a hard shell tablet or a capsule containing a binder, e.g., lactose or mannitol, a conventional filler, and a tabletting agent. Pharmaceutical compositions can also be administered via the parenteral route. Examples of parenteral dosage forms include aqueous solutions, isotonic saline or 5% glucose of the active agent, or other well-known pharmaceutically acceptable excipient. Solubilising agents well-known to those familiar with the art, can be utilized as pharmaceutical excipients for delivery of the anti-gastrointestinal cancer agent.

Injectable dosage forms may be formulated as liquid solutions or suspensions. Solid forms suitable for solution in, or suspension in, liquid prior to injection may also be prepared. The dosage form may also be emulsified. The one or more compounds derived from propolis, and when present the at least one additional anti-gastrointestinal cancer factor may be mixed with carriers such as, for example, water, saline, dextrose, glycerol, ethanol, or the like and combinations thereof.

Sustained-release preparations may be prepared incorporating one or more of the contemplated compounds. Suitable examples of sustained-release preparations include semi-permeable matrices of solid hydrophobic polymers containing one or more of the compounds, and when present the at least one additional anti-gastrointestinal cancer agent. The matrices may be in the form of shaped articles, e.g., films, or microcapsules. Non-limiting examples of sustained-release matrices include polyesters, hydrogels (for example, poly(2-hydroxyethyl- methacrylate), or poly(vinylalcohol)), polylactides (see US 3,773,919), copolymers of L-glutamic acid and ethyl-L-glutamate, non-degradable ethylene-vinyl acetate, and degradable lactic acid-glycolic acid copolymers such as the LUPRON DEPOT™ (injectable microspheres composed of lactic acid-glycolic acid copolymer and leuprolide acetate).

Described herein is a parenteral unit dosage form comprising one or more of the compounds specifically described herein, optionally with at least one additional therapeutic agent.

In various embodiments, the at least one additional therapeutic agent is an antibiotic, such as an aminoglycoside, such as amikacin, gentamicin, kanamycin, neomycin, netilmicin, streptomycin, tobramicin, or paromomycin; an ansamycin, such as geldanamycin, or herbimycin; a carbacephem, such as loracarbef; carbapenems, such
as, ertapenem, doripenem, imipenem/cilastatin, or meropenem; cephalosporins (first generation), such as cefadroxil, cefazolin, cefalotin or cefalothin, or cefalexin; cephalosporins (second generation), such as cefaclor, cefamandole, cefoxitin, cefprozil, or cefuroxime; cephalosporins (third generation), such
as cefixime, cefdinir, cefditoren, cefoperazone, cefotaxime, cefpodoxime, ceftazidime, ceftib uten, ceftizoxime, or ceftriaxone; cephalosporins (fourth generation), such as cefepime; cephalosporins (fifth generation), such as ceftobiprole; glycopeptides, such as teicoplanin, or vancomycin; macrolides, such
as azithromycin, clarithromycin, dirithromycin, erythromycin, roxithromycin, troleandomycin , telithromycin, or spectinomycin; monobactams, such as aztreonam; penicillins, such as amoxicillin, ampicillin, azlocillin, carbenicillin, cloxacillin, dicloxacillin, flucloxacillin, mezlo cillin, meticillin, nafcillin, oxacillin, penicillin, piperacillin, or ticarcillin; polypeptides, such as bacitracin, colistin, or polymyxin b; quinolones, such
as ciprofloxacin, enoxacin, gatifloxacin, levofloxacin, lomefloxacin, moxifloxacin, norfloxacin, or ofloxacin; sulfonamides, such as mafenide, sulfonamidochrysoidine (archaic), sulfacetamide, sulfadiazine, sulfamethizole, sulfanilimide
(archaic), sulfasalazine, sulfisoxazole, trimethoprim, or trimethoprim-sulfamethoxazole (co-trimoxazole) (tmp-smx); tetracyclines, such
as demeclocycline, doxycycline, minocycline, oxytetracycline, tetracycline; others such as arsphenamine, chloramphenicol, clindamycin, lincomycin, ethambutol, fosfomycin, fusidic acid, furazolidone, isoniazid, linezolid, metronidazole, mupirocin, nitrofurantoin, platensimyc in, pyrazinamide, quinupristin/dalfopristin, rifampicin (rifampin in
US), thiamphenicol, tinidazole, dapsone, clofazimine; or a cyclic lipopeptides, such as daptomycin, a glycylcycline, such as tigecycline, or an oxazolidinones, such as linezolid.

In other embodiments, the at least one additional therapeutic agent is an antifungal, such as a polyene antifungal, such as natamycin, rimocidin, filipin, nystatin, amphotericin B, candicin; imidazoles, such
as miconazole, ketoconazole, clotrimazole, econazole, bifonazole, butoconazole, fenticonazol e, isoconazole, oxiconazole, sertaconazole, sulconazole, or tioconazole; triazoles, such as fluconazole, itraconazole, isavuconazole, ravuconazole, posaconazole, voriconazole, or terconazole; thiazoles such as abafungin; allylamines, such
as terbinafine, amorolfine, naftifine, or butenafine; echinocandins, such as anidulafungin, caspofungin, or micafungin; others such as benzoic
acid, ciclopirox, tolnaftate, undecylenic acid, flucytosine or
5-fluorocytosine, griseofulvin, haloprogin, and sodium bicarbonate; or alternatives such as allicin, tea tree oil, citronella oil, iodine, lemon grass, olive leaf, orange oil, palmarosa oil, patchouli, lemon myrtle, neem seed oil, coconut oil, zinc, or selenium

Alternatively the agent is selected from any of those described herein.

The efficacy of a composition useful according to the invention can be evaluated both *in vitro* and *in vivo.* Briefly, in one embodiment the composition can be tested for its ability, to for example, inhibit neoplastic cell proliferation *in vitro.* For *in vivo* studies, the composition can be fed to or injected into an animal (e.g., a mouse) and its effects on cancer cell survival, proliferation, metastasis, or one or more symptoms of gastrointestinal cancer or associated disease or disorder are then assessed. Based on the results, an appropriate dosage range, frequency, and administration route can be determined.

The compositions useful herein may be used alone or in combination with one or more other anti-gastrointestinal cancer agents, or one or more additional therapeutic agents. The anti-gastrointestinal cancer agent or additional therapeutic agent may be or comprise a food, drink, food additive, drink additive, food component, drink component, dietary supplement, nutritional product, medical food, nutraceutical, medical device, medical supply, medicament or pharmaceutical. The anti-gastrointestinal cancer agent or additional therapeutic agent is preferably effective to attenuate one or more neoplastic diseases or disorders or one or more of the symptoms of one or more neoplastic diseases or disorders, or otherwise confer a benefit on the subject to whom it is administered. Preferred therapeutic agents include therapeutic food factors, immunogenic or immunostimulatory agents, wound healing agents, and the like.

It should be understood that the additional anti-gastrointestinal cancer or therapeutic agents listed above (both food based and pharmaceutical agents) may also be employed in a method described herein where they are administered separately, simultaneously or sequentially with a composition useful herein.

As will be appreciated, the dose of the composition administered, the period of administration, and the general administration regime may differ between subjects depending on such variables as the severity of symptoms of a subject, the type of disorder to be treated, the mode of administration chosen, and the age, sex and/or general health of a subject. However, by way of general example, from about 1 mg to about 5000 mg per kg body weight of a composition useful herein is administered, 1 mg to about 4000 mg per kg body weight of a composition useful herein is administered, 1 mg to about 3000 mg per kg body weight of a composition useful herein is administered, 1 mg to about 2000 mg per kg body weight of a composition useful herein is administered, 1 mg to about 1000 mg per kg body weight of a composition useful herein is administered, per administration or per day, preferably about 50 to about 1000 mg per kg, preferably per day. In one embodiment, the administration is of from about 0.05 mg to about 250 mg per kg body weight of a composition useful herein.

In various embodiments, sufficient composition is administered to deliver from about 0.001 mg to about 50 mg of at least one compound described herein per kg body weight, from about 0.001 mg to about 40 mg of propolis per kg body weight, from about 0.001 mg to about 30 mg of propolis per kg body weight, from about 0.001 mg to about 20 mg of propolis per kg body weight, from about 0.001 mg to about 10 mg of propolis per kg body weight, from about 0.001 mg to about 5 mg of propolis per kg body weight, from about 0.001 mg to about 1 mg of propolis per kg body weight, from about 0.001 mg to about 0.5 mg of propolis per kg body weight, from about 0.001 mg to about 0.1 mg of propolis per kg body weight, or from about 0.001 mg to about 0.05 mg of propolis per kg body weight, per administration or per day.

It should be appreciated that administration may include a single dose, such as a single daily dose, or administration of a number of discrete divided doses as may be appropriate. It should be understood that a person of ordinary skill in the art will be able without undue experimentation, having regard to that skill and this disclosure, to determine an effective dosage regime (including dose and timing of administration) for a given condition.

When used in combination with another anti-gastrointestinal cancer agent or therapeutic agent, the administration of a composition useful herein and the other anti-gastrointestinal cancer agent or therapeutic agent may be simultaneous or sequential. Simultaneous administration includes the administration of a single dosage form that comprises all components or the administration of separate dosage forms at substantially the same time. Sequential administration includes administration according to different schedules, preferably so that there is an overlap in the periods during which the composition useful herein and other therapeutic agent are provided.

Additionally, it is contemplated that a composition in accordance with the invention may be formulated with additional active ingredients which may be of benefit to a subject in particular instances. For example, therapeutic agents that target the same or different facets of the disease process may be used.

Accordingly, "foods and beverages comprising anti-gastrointestinal cancer compositions" described herein can be used for general foods and health food. Since the anti-gastrointestinal cancer compositions described herein mask the taste of the compounds described herein, or of propolis, they can be eaten as they are or in the form of powder. They can be used as an ingredient or raw material for cake, biscuit, cookie, chocolate, sweets and other confectionary, including drops or chewing gum. The compositions of the invention may be added to water as a drink, can be used as sweetener for beverages such as milk, tea, coffee, hot chocolate, etc., and as an ingredient or raw material for fruit juice beverages, sports drink, etc.

Exemplary compositions described herein, and methods for preparing such compositions will now be described with reference to the following examples.

### EXAMPLES

Example 4 relating to 1,1-dimethylallyl caffeic acid, example 5 relating to 3-methyl-3-butenyl caffeic acid, example 9 relating to benzyl ferulate, and example 10 relating to benzyl isoferulate fall in the scope of the claims.

### Example 1

This example describes an assessment of the anti-gastrointestinal cancer activity of fractions of propolis produced by preparative chromatography. This study was performed using proliferation assays in the colon cancer adenocarcinoma cell line, DLD-1.

### Materials and methods

Test samples shown below in Table 2 were assessed for their ability to modulate the viability and proliferation of human colorectal adenocarcinoma cells (DLD-1) as assessed by the MTT assay. A positive control, 5-fluorouracil (5-FU) was included in addition to an unsupplemented cell control (negative control) in the study. The test samples were obtained by fractionation of propolis tincture.

### Production of propolis tincture fractions by column chromatography

Fractionation was carried out using a glass column packed with Merck Lichroprep CI8 reversed phase stationary phase (16 x 4 cm) which had been washed with methanol (MeOH, 200 mL) and equilibrated with 20% aqueous EtOH (500 mL). Propolis (5.446 g) dissolved in ethanol (EtOH, 5 mL) was loaded onto the top of the column using a piston pump. Elution was carried out as a stepped gradient (250 mL) consisting of 20%, 30%, 40%, 50%, 60%, 70%, 80%, and 90% aqueous EtOH, followed by two 100% EtOH steps, then elution with 2-propanol (IPA),ethyl acetate (EtOAc), acetone, and chloroform (CHCl₃). Solvent from the various fractions was removed under vacuum on a rotary evaporator followed by freeze drying overnight. The two 100% EtOH fractions were pooled as were the remaining four non-polar fractions (IPA, EtOAc, acetone, and CHCl₃) for biological assay work due to their relatively low masses.

The fractions are shown in Table 2 according to the percentage of ethanol used in the elution step from the column, e.g. 20%, 30%, 40%, 50%, 60%, 70%, 80%, and 90% aqueous EtOH, and 100% EtOH.

### Materials and methods for the DLD-1 colon cancer anti-proliferative assay

Test samples shown below in Table 2 were assessed for their ability to modulate the viability and proliferation of human colorectal adenocarcinoma cells (DLD-1) as assessed by the MTT assay. A positive control, 5-fluorouracil (5-FU) was included in addition to an unsupplemented cell control (negative control) in the study.

**Table 2. Test Samples**

| **Sample #** | **Test Sample ID** | **Elution solvent** | **Fraction mass (g)** | **% of recovered mass** |
|---|---|---|---|---|
| 1 | Propolis tincture | | | |
| 2 | Propolis fraction 1 | 20% EtOH | 0.1127 | 2.07 |
| 3 | Propolis fraction 2 | 30% EtOH | 0.0658 | 1.21 |
| 4 | Propolis fraction 3 | 40% EtOH | 0.2408 | 4.42 |
| 5 | Propolis fraction 4 | 50% EtOH | 0.5022 | 9.22 |
| 6 | Propolis fraction 5 | 60% EtOH | 2.2333 | 41.01 |
| 7 | Propolis fraction 6 | 70% EtOH | 0.9843 | 18.07 |
| 8 | Propolis fraction 7 | 80% EtOH | 0.4697 | 8.62 |
| 9 | Propolis fraction 8 | 90% EtOH | 0.2413 | 4.43 |
| 10 | Propolis fraction 9 | 100% EtOH (2) | 0.3018 | 5.54 |
| 11 | Propolis fraction 10 | IPA, EtOAc, acetone, and CHCl₃ | 0.042 | 0.77 |

### Description of test materials and test methods

Human colorectal adenocarcinoma epithelial cell lines were revived from cryostorage and cultured in the presence of the test and reference samples. The culture conditions for the cells were those described by the supplier of the cells (ATCC). An MTT assay was then performed on the cultures to determine the effect of the samples on the cell proliferation.

The methodology was based on the procedures reported by:
Smolka, AJ, Goldenring, JR, Gupta, S and Charles E Hammond, CE. Inhibition of gastric H,K-ATPase activity and gastric epithelial cell IL-8 secretion by the pyrrolizine derivative ML 3000. (2004). BMC Gastroenterology. 4: 4.
Chailler, P and Menard, D (2005). Establishment of Human Gastric Epithelial (HGE) Cell Lines Exhibiting Barrier Function, Progenitor, and Prezymogenic Characteristics. Journal of Cellular Physiology 202: 263-274.
Trainer, D.L., Kline, T., McCabe, F.L., Faucette, L.F., Field, J., Chaikin, M., Anzano, M., Rieman, D., Hoffstein, S., Li, D-J., Gennaro, D., Buscarino, C., Lynch, M., Poste, G. And Greig, R. (1988). Biological characterization and oncogene expression in human colorectal carcinoma cells lines. International Journal of Cancer 41: 287-296.

### Sample Preparation

Working solutions were prepared by dissolving the test fractions in 15% ethanol (ETOH)/HBSS to a concentration of 2 mg/mL solids.

### Experimental Procedures

### Characterisation of the Test System

1. Human colorectal adenocarcinoma cells (ATCC CCI-221, DLD-1).
2. Penicillin-streptomycin solution: 10000units/mL penicillin, 10 mg/mL streptomycin in 0.9% NaCl (Sigma Cat #P-0781). Stored at -20°C.
3. DMEM culture medium (Invitrogen Cat # 12100-046) Stored at-20°C.
4. Trypsin-EDTA solution: 0.25% Trypsin/EDTA, Invitrogen Cat#15400054 (x10 in stock).
5. Phosphate buffered saline (PBS) (Prepared by TBL).
6. Hanks Balanced Salt Solution (HBSS). (GIBCO Cat No. 14185-052). Stored at 4°C.
7. Foetal Bovine Serum (GIBCO Cat # 10091-148). Stored at -20°C.
8. MTT Reagent: 100 mg/vial, (SIGMA Cat. No. M-2128) dissolved in PBS at 10 mg/mL and stored at -20 °C. 5 mg/mL MTT solution were prepared in PBS and stored at 4 °C as working solution.
9. MTT lysis buffer: 10 % sodium dodecyl sulphate (SDS)/45% Dimethyl Formamide (20 g SDS was dissolved in 100 mL of double-distilled water (DDW), and 90 mL of Dimethyl Formamide added to the SDS solution). The pH was adjusted to 4.7 by glacial acetic acid, and DDW added up to a final volume of 200 mL.
10. 5-Fluorouracil (5-FU), (Sigma Cat. No. F-6627). Two working solutions were prepared at 150ng/mL and 75ng/mL, dissolved in 15% Ethanol/HBSS. Final concentrations were 7.50ng/mL and 3.75ng/mL.

### Medium preparation

The medium for the propagation of the colorectal adenocarcinoma (DLD-1) cells was DMEM, supplemented with penicillin-streptomycin solution (10mL per litre). FBS was added just before use to give 10% w/v.

### Culturing of cells

1. The human colorectal adenocarcinoma cells (DLD-1) obtained from the American Type Culture Collection USA were revived from cryostorage.
2. Following initial propagation using the media described above (see Medium Preparation), the culture was subcultured using the trypsin-EDTA as follows. The media was removed and 5mL of the trypsin-EDTA solution added and incubated at 37°C for 5 min or until all the cells had detached. The trypsin was neutralised by adding an equal volume of DMEM medium and the culture centrifuged at 300g (1200rpm) for 5 min at 4°C.
3. The supernatant was decanted and the cell pellets resuspended in medium DMEM, FBS (10%), penicillin (100units/mL), streptomycin (100µg/mL). The cells were cultured at 37°C in 5% CO₂/95% air.
4. After reaching confluence, the cells were detached using trypsin-EDTA and centrifuged as described in 2 above.
5. The supernatants were discarded and the cells resuspended in DMEM and supplements as described in 3 above at 1.0 x 10⁴ cells per mL.
6. Into each well of three 96 well plates, 180µl of the cells (1,800 cells/well) or medium was added. The plates were incubated at 5% CO₂/95% air at 37°C for 48 h which was sufficient to allow the cells to adhere.
7. To each well, 20µl of each of the test samples or positive control was added. For the 'medium' or 'cells only' controls, 20µl of 15%ETOH/HBSS was added to each well. Each sample was assessed in replicates of 6, while the controls on each of three plates were assessed in replicates of 9 (combined triplicates). The final concentration of the sample in each well was 200 µg/mL.
8. The total volume in each well was 200µl.
9. The plates were incubated at 37°C in 5% CO₂/95% air for 19 h.

### Cell Proliferation Assay

1. On completion of the incubation, 20 µl of MTT working solution (5 mg/mL) was added to all wells and incubated for 3-4 hr at 37° C in 5% CO₂/95% air.
2. During this incubation period it was noted that a strong colour unexpectedly appeared in some of the wells of a number of samples, in both the Cells plus Sample wells and the Sample plus Medium Blank wells. At the end of this period the supernatants were removed from each well and each well was gently washed with HBSS twice, to remove the purple colour, before adding the MTT lysis buffer as described in Step 3 below.
3. 100 µl of MTT lysis buffer was then added and the plates incubated overnight at 37°C in 5% CO₂/95% air. The plates were centrifuged at 1200rpm for 10 minutes to pellet any remaining insoluble material. From each well 200µl aliquots were transferred to fresh 96 well plates. The plates were read by a VersaMax microplate reader at 570 nm.
4. Results were expressed as the percentage proliferation of cells cultured in the presence of the sample in comparison to the cells only control. The blank reading was subtracted from all wells as a background reading.

### Results

A summary of the effects of controls and test samples on the proliferation of the cells is presented in Table 3 below.
- At 200 µg/mL the strongest inhibitors of proliferation of colon cancer cells were Propolis Fraction #1 (68.1%), Propolis Fraction #2 (72.8%), Propolis Fraction #5 (41.0%) and Propolis Fraction #8 (72.0%).
- At 200 µg/mL Propolis Tincture inhibited the proliferation of colon cancer cells by 32.6%.

**Table 3. Effects of the test samples on the proliferation of DLD-1 cells.**

| **Sample ID** | **Mean (OD 570nm)** | **SEM** | **pValues (<0.05)** | **% Inhibition** |
|---|---|---|---|---|
| Cells Only | 0.7047 | 0.0252 | 1.0 | 0.00 |
| Cells - 5-FU (7.50ng/ml) (reference) | 06575 | 0.0172 | NS | 6.69 |
| Cells - S#1. Propolis Tincture | 0.4750 | 0.0207 | 2.0E-5 | 32.59 |
| Cells - S#2. Propolis Fraction #1 | 0.2246 | 0.0297 | 2.0E-8 | 68.13 |
| Cells - S#3. Propolis Fraction #2 | 0.1919 | 0.0250 | 2.0E-8 | 72.77 |
| Cells - S#4. Propolis Fraction #3 | 0.5359 | 0.0153 | 2.3E-4 | 23.95 |
| Cells - S#5. Propolis Fraction #4 | 0.5779 | 0.0203 | 3.2E-03 | 17.99 |
| Cells - S#6. Propolis Fraction #5 | 0.4159 | 0.0172 | 1.2E-6 | 40.97 |
| Cells - S#7. Propolis Fraction #6 | 0.6488 | 0.0197 | NS | 7.93 |
| Cells - S#8. Propolis Fraction #7 | 0.6037 | 0.0135 | 9.2E-3 | 14.32 |
| Cells - S#9. Propolis Fraction #8 | 0.1970 | 0.0152 | < 1E-3 | 72.04 |
| Cells - S#10. Propolis Fraction #9 | 0.5932 | 0.0194 | 7.0E-3 | 15.82 |
| Cells - S#11. Propolis Fraction #10 | 0.6375 | 0.0309 | NS | 9.54 |

Further studies were conducted to determine the compounds present in the most active fractions. These studies are described in Example 2.

### Example 2

This example describes an assessment of the anti-gastrointestinal cancer activity of compositions described herein, compared to pure compound standards. This study was performed using proliferation assays in the colon cancer adenocarcinoma cell line, DLD-1 as described in Example 1.

### Preparative HPLC

The 20%, 30%, 60%, and 90% aqueous EtOH elution fractions produced for Example 1 were further fractionated by preparative HPLC. Dried 20% and 30% EtOH fractions were dissolved in EtOH/H₂O (1:1), while the 60% and 90% EtOH fractions were dissolved in neat EtOH. Each solution was chromatographed by preparative HPLC on a Phenomenex Synergi 4|i,-RP Max 80A 250 x 30 mm C-12 column using a Gilson 321 preparative pump and Agilent 1100 series diode array detector. Injection volumes of between 0.5 and 1.5 mL were employed. A flow rate of 20 mL/min. was employed. For the 20%, 30%, and 60% EtOH fractions, an initial solvent composition of 70% water (containing 0.1% trifluoroacetic acid [TFA] vol/vol.) and 30% MeOH (containing 0.1% TFA vol./vol.) was used. The solvent composition was held constant for 10 minutes at the initial conditions before the MeOH concentration was increased linearly to 40% over 8 minutes, and held at this composition for 5 minutes before being increased to 80% over 32 minutes then to 100% over 5 minutes.

The chromatography was carried out at room temperature, being ca. 18 °C. Fractions were collected manually with online detection carried out at 268 nm (for flavonoids) and 327 nm (for caffeic acid derivatives). Analytical HPLC was carried out on all collected fractions and those showing a single compound or a highly purified compound had solvent removed under vacuum on a rotary evaporator, were weighed, and prepared for biological assay. Fractions from the 20% and 30% series not submitted for further assay were combined to form a 'non-peak' 20% and 30% fraction for biological assay to confirm that major activity was due to observed peaks rather than any material not observed at the chosen wavelengths. Two of the 60% fractions, being 60% EtOH F8 and 60% EtOH F9, required further chromatography (see Sephadex LH20 Chromatography below). The 90% EtOH fraction was also subjected to chromatography. However, the solvents used and gradient profiles were modified for chromatography of this fraction. Solvents employed here were 80% aqueous MeOH (containing 0.1% TFA) and EtOAc/MeOH (4:1 vol/vol). The initial eluent composition consisted of the 80% aqueous methanol. The solvent composition was held for 5 minutes at the initial conditions before the EtOAc/MeOH solvent concentration was increased linearly to 100% over 35 minutes. Analysis of the 90% EtOH fraction by analytical HPLC showed that the main components of this fraction were quite non polar, eluting late in the chromatogram, and showed minimal UV absorption at the wavelengths used for analysis on other propolis fractions, i.e., 268 and 327 nm. However, evaporative light scattering detection (ELSD) did reveal a complex mix of components. Due to the destructive nature of ELSD preparative HPLC fractions were collected manually with online detection carried out at 210 nm. As the chromatography did not produce distinct isolated peaks but rather broad rises and falls in the baseline four fractions were collected during the run and all were prepared for biological assay.

### Sephadex LH20 Chromatography

Analytical HPLC analysis of the fractions 60% EtOH F8 and F9 derived from the preparative HPLC work as noted above showed these fractions to contain several closely eluting compounds. These fractions were subjected to further chromatography, as follows.

Each of these fractions was applied to the top of a glass column containing Sephadex LH20 stationary phase (3 x 42 cm) which had been previously equilibrated with 70% aqueous MeOH (containing 0.1% TFA vol/vol). Fractions were collected on a time basis using a fraction collector, with individual fraction volumes of about 7 mL. Fractions were examined by analytical HPLC and those containing single compounds or a highly purified compound were combined and prepared for biological assay. The remaining fractions from each series were combined and prepared for biological assay as a 'non-peak' fraction.

### Materials and methods for the DLD-1 colon cancer anti-proliferative assay

Test samples shown below in Table 4 were assessed for their ability to modulate the viability and proliferation of human colorectal adenocarcinoma cells (DLD-1) as assessed by the MTT assay. A positive control, 5-fluorouracil (5-FU) was included in addition to an unsupplemented cell control (negative control) in the study. The DLD-1 anti-proliferation assay was performed as described in Example 1.

**Table 4. Test Samples**

| **Sample No** | **Test Sample ID** | **Sample No** | **Test Sample ID** |
|---|---|---|---|
| #1 | 20% F1 | #19 | 60% F8 (54-60) |
| #2 | 20% F6 | #20 | 60% F9 (26-29) |
| #3 | 20% F8 | #21 | 60% F9 (34-36) |
| #4 | 20% F10 | #22 | 60% F9 (39-46) |
| #5 | 20% F11 | #23 | 60% F9 (49-51) |
| #6 | 20% non fraction | #24 | 60% F9 (54-57) |
| #7 | 30% F5 | #25 | 60% F9 (58-64) |
| #8 | 30% F8 | #26 | 60% non fraction |
| #9 | 30% F10 | #27 | 60% F10 |
| #10 | 30% F11 | #28 | 90% F1 |
| #11 | 30% F12 | #29 | 90% F2 |
| #12 | 30% non fraction | #31 | 90% F4 |
| #13 | 60% F7 | #32 | Chrysin (reference) |
| #14 | 60% F8(27-30) | #33 | CAPE (Caffeic acid phenethyl ester) |
| #15 | 60% F8 (35-40) | #34 | Trans-Cinnamic acid (reference) |
| #16 | 60% F8 (41-43) | #35 | Chrysin 7-methyl ether (Tectochrysin) (reference) |
| #17 | 60% F8 (44-50) | #36 | Galangin (reference) |
| #18 | 60% F8 (52-53) | #37 | Pinocembrin (reference) |
| #19 | 60% F8 (54-60) | #38 | Pinocembrin 7-methyl ether (reference) |
| #20 | 60% F9 (26-29) | #39 | Caffeic acid (reference) |

The samples were dissolved as working solutions at 2 mg/mL in 15% EtOH in HBSS, except for Sample #1, which was at 1 mg/mL in 15% ETOH/HBSS. In the assay the final concentration of the samples was 200 µg/mL with a final EtOH concentration of 1.5%, except for Sample #1, which was at 100 µg/mL with a final EtOH concentration of 1.5%.

### Results and Discussion

### Summary

- The majority of the samples tested were inhibitors of the proliferation of the DLD-1 colon cancer cells.
- None of the samples had a stimulatory effect.
- Of the propolis fractions, the strongest inhibitors were (inhibitory level in brackets):
   ∘ # 3 20% F8 (95.2%)
   ∘ # 8 30% F8 (96.1%)
   ∘ # 14 60% F8 (27-30) (95.0%)
   ∘ # 17 60% F8 (44-50) (90.4%)
   ∘ # 18 60% F8 (52-53) (90.4%)
- Several samples had no or negligible effect on the growth of the colon cancer. These included
   ∘ # 1 20% F1 (0.4% inhibition)
   ∘ # 9 30% F10 (3.2% stimulation)
   ∘ # 10 30% F11 (7.5% inhibition)
   ∘ # 38 Pinocembrin 7-methyl ether (2.7% inhibition) (reference)

A summary of the effects of the positive control and test samples on the proliferation of the cells after 24 hours incubation is presented in Table 5 below.

**Table 5. Effect of the test samples on the proliferation of DLD-1 cells.**

| **Sample ID** | **Mean OD (570nm)** | **SEM** | **P Values (<0.05)** | **% Inhibition** |
|---|---|---|---|---|
| Cells only | 0.3239 | 0.0135 | NS | 0.00 |
| Cells + 5-FU (reference) | 0.3023 | 0.0154 | NS | 6.66 |
| Cells + 5-FU (reference) | 0.2689 | 0.0116 | 5.3E-3 | 16.96 |
| Cells + S#1. 20% F1 | 0.3225 | 0.0314 | NS | 0.42 |
| Cells + S#2. 20% F6 | 0.2085 | 0.0209 | 2.0E-4 | 35.63 |
| Cells + S#3. 20% F8 | 0.0156 | 0.0022 | 3.7E-9 | 95.19 |
| Cells + S#4. 20% F10 | 0.2152 | 0.0230 | 1.2E-3 | 33.56 |
| Cells + S#5. 20% F11 | 0.1539 | 0.0146 | 4.6E-5 | 52.49 |
| Cells + S#6. 20% non fraction | 0.2295 | 0.0246 | 2.0E-3 | 29.13 |
| Cells + S#7. 30% F5 | 0.2316 | 0.0244 | 2.3E-3 | 28.50 |
| Cells + S#8. 30% F8 | 0.0126 | 0.0006 | <1E-10 | 96.12 |
| Cells + S#9. 30% F10 | 0.3344 | 0.0081 | NS | -3.24 |
| Cells + S#10. 30% F11 | 0.2995 | 0.0040 | NS | 7.53 |
| Cells + S#11. 30% F12 | 0.2640 | 0.0065 | 8.3E-3 | 18.47 |
| Cells + S#12. 30% non fraction | 0.2784 | 0.0053 | 3.4E-2 | 14.05 |
| Cells + S#13. 60% F7 | 0.1299 | 0.0062 | 3.6E-8 | 59.89 |
| Cells + S#14. 60% F8 | 0.0163 | 0.0021 | 3E-10 | 94.96 |
| Cells + S#15. 60% F8 | 0.1233 | 0.0118 | 5.0E-8 | 61.92 |
| Cells + S#16. 60% F8 | 0.1332 | 0.0120 | 1.1E-7 | 58.86 |
| Cells + S#17. 60% F8 | 0.0310 | 0.0010 | 7.1E-9 | 90.43 |
| Cells + S#18. 60% F8 | 0.0312 | 0.0021 | 7E-10 | 90.37 |
| Cells + S#19. 60% F8 | 0.0475 | 0.0029 | 2E-10 | 85.33 |
| Cells + S#20. 60% F9 | 0.1369 | 0.0082 | 7.6E-8 | 57.73 |
| Cells + S#21. 60% F9 | 0.2544 | 0.0296 | 2.5E-2 | 21.44 |
| Cells + S#22. 60% F9 | 0.1838 | 0.0082 | 3.2E-6 | 43.25 |
| Cells + S#23. 60% F9 | 0.1013 | 0.0036 | 4.2E-9 | 68.73 |
| Cells + S#24. 60% F9 | 0.0485 | 0.0064 | 1.9E-9 | 85.02 |
| Cells + S#25. 60% F9 | 0.1205 | 0.0088 | 2.6E-8 | 62.81 |
| Cells + S#26. 60% non fraction | 0.1610 | 0.0079 | 4.6E-7 | 50.28 |
| Cells + S#27. 60% F10 | 0.0715 | 0.0056 | 8E-10 | 77.93 |
| Cells + S#28. 90% F1 | 0.1909 | 0.0137 | 1.3E-5 | 41.06 |
| Cells + S#29. 90% F2 | 0.2528 | 0.0106 | 3.3E-3 | 21.96 |
| Cells + S#31. 90% F4 | 0.2776 | 0.0118 | 4.2E-2 | 14.30 |
| Cells + S#32. Chrysin (reference) | 0.1233 | 0.0099 | 3.7E-8 | 61.92 |
| Cells + S#33. CAPE (Caffeic acid phenethyl ester) | 0.0777 | 0.0071 | 7.4E-8 | 76.01 |
| Cells + S#34. Trans-Cinnamic acid (reference) | 0.2390 | 0.0136 | 1.1E-3 | 26.21 |
| Cells + S#35. Chrysin 7-methyl ether (Tectochrysin) (reference) | 0.2285 | 0.0160 | 5.6E-40.0005590171 | 29.45 |
| Cells + S#36. Galangin (reference) | 0.1411 | 0.0038 | 0.67E-8 | 56.45 |
| Cells + S#37. Pinocembrin (reference) | 0.0196 | 0.0018 | 4.3E-9 | 93.95 |
| Cells + S#38. Pinocembrin 7-methyl ether (reference) | 0.3151 | 0.0090 | NS | 2.71 |
| Cells + S#39. Caffeic acid (reference) | 0.0113 | 0.0016 | 3.0E-9 | 96.52 |

### Compound Identification

The fractions that were the strongest inhibitors of colon cancer cell proliferation were further analysed to identify compounds present in these fractions. The compounds identified in the active fractions are presented in Table 6. The method used to identify each compound is detailed below.

### 5-phenylpenta-2,4-dienoic acid

The HPLC on-line UV-Visible absorption spectrum and low resolution LCMS m/z data were consistent with the structure of 5-phenylpenta-2,4-dienoic acid, which has been previously reported to be present in New Zealand sourced propolis (Markham et al, 1996. HPLC and GC-MS identification of the major organic constituents in New Zealand propolis. Phytochemistry, 42(1): 205-211). The structure was confirmed by co-chromatography on HPLC with an authentic standard.

### 3-methyl-3-butenyl caffeic acid

The HPLC on-line UV-Visible absorption spectrum and low resolution LCMS m/z data were consistent with the structure of 3-methyl-3-butenyl caffeic acid, which has been previously reported to be present in New Zealand sourced propolis (Markham et al, 1996). Additionally, HRMS, 'H- and ^{l3}C NMR data are consistent with published data for 3-methyl-3-butenyl caffeic acid.

### 1,1-dimethylallylcaffeic acid

The HPLC on-line UV-Visible absorption spectrum and low resolution LCMS m/z data were consistent with the structure of 1,1-dimethylallylcaffeic acid, which has been previously reported to be present in New Zealand sourced propolis (Markham et al, 1996). The structure was confirmed by co-chromatography on HPLC with an authentic standard. Additionally, HRMS, 'H- and ^{l3}C NMR data are consistent with published data for 1,1-dimethylallylcaffeic acid.

### Pinobanksin-3-acetate

The HPLC on-line UV-Visible absorption spectrum and low resolution LCMS m/z data were consistent with the structure of pinobanksin-3-acetate, which has been previously reported to be present in New Zealand sourced propolis (Markham et al, 1996). A laboratory reference sample was also available for comparison.

### Pinostrobin chalcone

Pinostrobin chalcone was isolated from crude propolis. The isolation involved several chromatographic steps; two on silica gel and a final clean up step using Sephadex LH-20. The main yellow compound from this final step was collected and shown to be pure pinostrobin chalcone by comparison of NMR and UV-Visible spectroscopic data with literature values (Malek, S.N.A.; Phang, C.W.; Ibrahim, H.; Abdul Wahab, N.; Sim, K.S. Phytochemical and Cytotoxic Investigations of Alpinia mutica Rhizomes. Molecules 2011, 16, 583-589.).

### Tectochrysin

The HPLC on-line UV-Visible absorption spectrum and low resolution LCMS m/z data were consistent with the structure of tectochrysin, which has been previously reported to be present in New Zealand sourced propolis (Markham et al, 1996).

### Benzyl ferulate and Benzyl isoferulate

The esters present in the S#23 60% F9 fraction were identified as a mixture of benzyl ferulate and benzyl isoferulate. Identification was on the basis of small scale hydrolysis of the fractions which yielded benzyl alcohol and a mixture of ferulic and isoferulic acids. The hydrolysis products were identified from comparison of HPLC data (retention times and on-line UV-VIS spectra) with that from reference compounds.

**Table 6. Compounds identified in active fractions**

| **Sample ID** | **Compounds identified in sample** |
|---|---|
| #3 20% F8 | caffeic acid |
| #8 30% F8 | caffeic acid |
| #13 60% F7 | 5-phenylpenta-2,4-dienoic acid |
| #14 60% F8 (27-30) | 1,1-dimethylallylcaffeic acid |
| #15 60% F8 (35-40) | 3-methyl-3-butenyl caffeic acid (major component) 1,1-dimethylallylcaffeic acid (minor component) pinobanksin-3-acetate (minor component) |
| #16 60% F8 (41-43) | pinobanksin-3-acetate |
| #17 60% F8 (44-50) | caffeic acid phenyl ester (CAPE) pinoban ksi n-3-acetate pinocembrin |
| #18 60% F8 (52-53) | Pinocembrin (major component) benzyl caffeate (minor component) |
| #19 60% F8 (54-60) | Pinocembrin (minor component) benzyl caffeate (major component) |
| #20 60% F9 (26-29) | cinnamyl caffeate |
| #22 60% F9 (39-46) | chrysin |
| #23 60% F9 (49-51) | Benzyl ferulate and benzyl isoferulate |
| #24 60% F9 (54-57) | Pinostrobin chalcone |
| #25 60% F9 (58-64) | galangin |
| #27 60% F10 | tectochrysin |

### Discussion

These data support the efficacy of compositions of the invention in inhibiting the proliferation of colorectal cell proliferation.

A selection of the most bioactive compounds from this Example were either purified, synthesized or used as a genuine pure standard in the following examples. Pinocembrin and p-coumaric acid were also tested as additional positive and negative controls on the basis of their strong and weak activity respectively

### Example 3

This example describes an assessment of the anti-gastrointestinal cancer activity of 5-phenylpenta-2,4-dienoic acid (used as a reference), a compound isolated from NZ-source European-type propolis as fraction S#14 60% F7(Table 6, Example 2). This study was performed using proliferation assays for the human colon cancer adenocarcinoma cell line, DLD-1; human colon cancer cell line, HCT-116; human gastric carcinoma cell line, NCI-N87; and human oesophageal squamous cell carcinoma cell line, KYSE-30.

The general method used for Examples 3 - 13 is shown below.

### Materials and methods for the gastro-intestinal cancer anti-proliferative assays

5-phenylpenta-2,4-dienoic acid was assessed for its ability to modulate the viability and proliferation of human colorectal adenocarcinoma cells (DLD-1); human colon cancer cell line (HCT-116); human gastric carcinoma cell line (NCI-N87); and human oesophageal squamous cell carcinoma cell line (KYSE-30) as assessed by the MTT assay. The positive control, 5-fluorouracil (5-FU) was included at three concentrations; in addition to an unsupplemented cell control as a negative control in the study. The 5-phenylpenta-2,4-dienoic acid was a genuine standard with chemical structure confirmed by NMR and MS.

### Test materials and test methods

The four human gastrointestinal carcinoma cell lines were revived from cryostorage and cultured in the presence of the test and reference samples. An MTT assay was then performed on the cultures to determine the effect of the samples on the cell viability and proliferation.

The methodology is based on the procedures reported in the following references:
Smolka, AJ, Goldenring, JR, Gupta, S and Charles E Hammond, CE. Inhibition of gastric H,K-ATPase activity and gastric epithelial cell IL-8 secretion by the pyrrolizine derivative ML 3000. BMC Gastroenterology. Published 10 February 2004.
Chailler, P and Menard, D (2005). Establishment of Human Gastric Epithelial (HGE) Cell Lines Exhibiting Barrier Function, Progenitor, and Prezymogenic Characteristics. Journal of Cellular Physiology 202: 263-274.
Trainer, D.L. et al (1988). Biological characterization and oncogene expression in human colorectal carcinoma cells lines. International Journal of Cancer 41: 287-296

The culture conditions for the cells are those provided by the supplier of the cells (ATCC and ECACC).

### Sample Preparation

The test sample 5-phenylpenta-2,4-dienoic acid, and all other test samples in Examples 4 - 12 (except tectochrysin) were initially dissolved in pure ethanol to a concentration of around 13.35mg/ml. Tectochrysin was initially dissolved in triethylene glycol mono-methyl ether to the same concentration. Working solutions were then prepared from the stock solutions by dilution to 15% ethanol/Hanks Balanced Salt Solution (ETOH)/HBSS to a concentration of 2 mg/mL solids. In the assay the final concentration of the samples was 200µg/ml with a final EtOH concentration of 1.5%. Final concentrations of test compounds and propolis prepared as above are as noted in Example 3.

### Experimental Procedures

### Characterisation of the Test System

1. Human colorectal adenocarcinoma cells (ATCC CCI-221, DLD-1)(ATCC, Bethesda, MD, USA)
2. Human gastric carcinoma cells (ATCC CRL-5822, NCI-N87)(ATCC, Bethesda, MD, USA)
3. Human oesophageal squamous cell carcinoma (ECACC, KYSE-30)(Sigma Aldrich, Auckland, NZ)
4. Human colon carcinoma cells (ECACC HCT-116)(Sigma Aldrich, Auckland, NZ)
5. Penicillin-streptomycin solution: 10000units/ml penicillin, 10mg/ml streptomycin in 0.9% NaCl (Sigma Cat #P-0781). Stored at -20°C.
6. For DLD-1 cells: DMEM culture medium (Invitrogen Cat # 12100-046). Stored at 4°C.
7. For NCI-N87 cells: RPMI-1640 medium modified to contain 2 mM L-glutamine, 10 mM HEPES, 1 mM sodium pyruvate, 4500 mg/L glucose, and 1500 mg/L sodium bicarbonate (Sigma R6504). Stored at 4°C.
8. For KYSE-410 cells: RPMI-1640 medium modified to contain 2 mM L-glutamine (Sigma R6504). Stored at 4°C.
9. For HCT-116 cells: McCoy's 5A medium modified to contain 2 mM L-glutamine (Sigma M48792). Stored at 4°C.
10. Trypsin-EDTA solution: 0.25% Trypsin/EDTA, Invitrogen Cat#15400054 (x10 in stock).
11. Phosphate buffered saline (PBS) (Prepared by TBL).
12. Hanks Balanced Salt Solution (HBSS). (GIBCO Cat No. 14185-052). Stored at 4°C.
13. Foetal Bovine Serum (GIBCO Cat # 10091-148). Stored at-20°C.
14. MTT Reagent: 100 mg/vial, (SIGMA Cat. No. M-2128) dissolved in PBS at 10 mg/ml and stored at -20 °C. 5mg/ml MTT solution will be prepared in PBS and stored at 4 °C as working solution.
15. MTT lysis buffer: 10 % sodium dodecyl sulphate (SDS)/45% Dimethyl Formamide (20 g SDS will be dissolved in 100 ml of double-distilled water (DDW), and 90 ml of Dimethyl Formamide will be added to the SDS solution). The pH will be adjusted to 4.7 by glacial acetic acid, and DDW added up to a final volume of 200 ml.
16. 5-Fluorouracil (5-FU), (Sigma Cat. No. F-6627). Three working solutions will be prepared at 19.5µg/ml. 6.5µg/ml and 1.95µg/ml dissolved in 15% Ethanol/HBSS. Final concentrations will be 1.95µg/ml (15µM), 0.65µg/ml (5µM) and 0.195µg/ml (1.5µM).

### Medium preparation

The medium for the propagation of the each of the cells lines is given above. Each medium was prepared following the ATCC/ECACC instructions and supplemented with penicillin-streptomycin solution (10ml per litre). FBS (at 10%) was added just before use.

### Culturing of cells

1. Each of the cell lines obtained from either the American Type Culture Collection USA, or the European Collection of Cell Cultures was revived from cryostorage.
2. Following initial propagation using the media described above (see Characterisation of Test System, and *Medium Preparation*), the culture was subcultured using trypsin-EDTA. The media was removed and 5ml of the trypsin-EDTA solution added and incubated at 37°C for 5 min or until all the cells have detached. The trypsin was neutralised by adding an equal volume of the relevant medium and centrifuging the suspension at 300g (1200rpm) for 5 min at 4°C.
3. The supernatant was decanted and the cell pellets resuspended in the relevant medium containing FBS (10%), penicillin (100units/ml), streptomycin (100µg/ml).
4. After reaching confluence, the cells were detached using trypsin-EDTA as described in 2 above and centrifuged.
5. The supernatants were discarded and the cells resuspended in the relevant medium and supplements as described in 3 above at 1.0 x 10⁴ cells per ml. A total volume of approximately 54mls of each cell suspension was required.
6. Into each well of six 96 well plates, 180µl of the cells (1,800 cells/well) or medium was added according to a pre-determined plate layout. The plates were incubated at 5% CO₂/95% air at 37°C for ∼48hrs to allow the cells to adhere.
7. To each well, 20µl of each of the test compounds or 5-FU was added as indicated in the plate layouts. For negative control wells labelled 'medium' or 'cells only', 20µl of 15%ETOH/HBSS was added. Each sample or control was assessed in replicates of 3 or 6.
8. The total volume in each well was 200µl.
9. The plates were incubated at 37°C in 5% CO₂/95% air for 24hr.

### Cell Proliferation Assay

1. On completion of the incubation, 20 µl of MTT working solution (5mg/ml) was added to all wells and incubated for 3-4 hr at 37° C in 5% CO₂/95% air. The plates were monitored every 30-60 minutes and if a few cells showed the presence of crystals then the lysis buffer was added as in Step 2 below.
2. 100 µl of MTT lysis buffer was then added and the plates incubated overnight at 37°C in 5% CO₂/95% air. The plates were centrifuged at 1200rpm for 10 minutes to pellet any remaining insoluble material. From each well 200µl was transferred to fresh 96 well plates. The plates were then read in a VersaMax microplate reader at 550 nm.
3. The blank reading was subtracted from all wells as a background reading. Results were expressed as the percentage proliferation of cells cultured in the presence of the sample in comparison to the cells only control.
4. A VersaMax 96-well plate reader was used to colorimetrically (at 550nm) assess the proliferation of the cells.
5. The percentage standard error of the mean was assessed and extreme outliers will be removed if the SEM% >15. Preliminary statistical significance will be assessed with an independent Student t-test at α ≤ 0.05 (with and without outliers).

### Observations regarding the test method

The culturing of the several cancer cells in the presence of the various test preparations and the 5-FU was conducted for 48 hours as the visual observation of the cell density of the negative controls of the DLD-1 and NCI-N87 after 24 hours suggested that the growth was not as rapid as anticipated. For consistency the time for the other cultures was set as the same.

The study plan required outliers among the replicates to be deleted from the calculations if the percentage standard error of mean (SEM) was greater than 15%. When a sample is a strong antagonist, the absorbance values are small. Consequently small changes among these mean that the variance from the mean expressed as a percentage is relatively large when compared with less inhibitory samples. This has resulted in some outliers in which virtually complete inhibition occurred being removed from the analysis.

### Results

The results of the cell proliferation assay for 5-phenylpenta-2,4-dienoic acid are shown in Table 7, along with results for the positive control 5-fluorouracil (5-FU) at three concentrations, and the negative control (cells only with medium and no test compound or positive control).In the Table, OD is Optical Density measured at 570 nm; SEM is the Standard Error associated with the Mean Optical Density value measured; p is the probability value that the measurement is statistically significant via the Student t-test, here taken to be < 0.05; % stim. is the percentage stimulation of proliferation compared to the negative control (test compound inactive); % inhibition is the percentage reduction of proliferation compared to the negative control, with a large number indicating the test compound has anticancer proliferation potential.

**Table 7. Antiproliferative activity of 5-phenylpenta-2,4-dienoic acid against 4 gastro-intestinal cell lines**

| Cell type | Sample | Mean OD (570nm) | SEM | p values (<0.05) | % stim. | % inhibition |
|---|---|---|---|---|---|---|
| DLD-1 | Cells only (n=6) | 0.4122 | 0.0104 | 1 | 0 | 0 |
| | Cells + 5FU (0.195 µg/ml, n=6) | 0.3029 | 0.0074 | 6.36E-06 | 0 | 26.53 |
| | Cells + 5FU (0.65 µg/ml, n=6) | 0.2584 | 0.0113 | 1.59E-06 | 0 | 37.31 |
| | Cells + 5FU (1.95 µg/ml, n=6) | 0.2409 | 0.0073 | 9.50E-08 | 0 | 41.56 |
| | Cells + 5-phenylpenta-2,4 dienoic acid (200 µg/ml, n=6) | 0.2329 | 0.015 | 1.91E-06 | 0 | 43.5 |
| HCT-116 | Cells only (n=6) | 0.5084 | 0.0321 | 1.00E+00 | 0 | 0 |
| | Cells + 5FU (0.195 µg/ml, n=6) | 0.336 | 0.019 | 9.50E-04 | 0 | 33.92 |
| | Cells + 5FU (0.65 µg/ml, n=6) | 0.234 | 0.015 | 1.50E-05 | 0 | 53.97 |
| | Cells + 5FU (1.95 µg/ml, n=6) | 0.2169 | 0.0037 | 4.10E-06 | 0 | 57.34 |
| | Cells + 5-phenylpenta-2,4 dienoic acid (200 µg/ml, n=6) | 0.3232 | 0.0175 | 4.90E-04 | 0 | 36.43 |
| NCI-N87 | Cells only (n=6) | 0.4417 | 0.025 | 1 | 0 | 0 |
| | Cells + 5FU (0.195 µg/ml, n=6) | 0.4085 | 0.0081 | > 0.05 | 0 | 7.52 |
| | Cells + 5FU (0.65 µg/ml, n=6) | 0.3656 | 0.0212 | 0.043 | 0 | 17.22 |
| | Cells + 5FU (1.95 µg/ml, n=6) | 0.3702 | 0.0055 | 0.019 | 0 | 16.19 |
| | Cells + 5-phenylpenta-2,4 dienoic acid (200 µg/ml, n=6) | 0.2537 | 0.0287 | 5.80E-04 | 0 | 42.56 |
| KYSE-30 | Cells only (n=6) | 0.6017 | 0.0223 | 1 | 0 | 0 |
| | Cells + 5FU (0.195 µg/ml, n=6) | 0.4588 | 0.0107 | 1.80E-04 | 0 | 23.75 |
| | Cells + 5FU (0.65 µg/ml, n=6) | 0.3647 | 0.0188 | 1.00E-05 | 0 | 39.38 |
| | Cells + 5FU (1.95 µg/ml, n=6) | 0.316 | 0.0045 | 1.90E-07 | 0 | 47.48 |
| | Cells + 5-phenylpenta-2,4 dienoic acid (200 µg/ml, n=6) | 0.3758 | 0.0065 | 2.00E-06 | 0 | 37.55 |

### Discussion

5-phenylpenta-2,4-dienoic acid (used as a reference) was active against all four cancer cell lines resulting in inhibition of proliferation of human colon adenocarcinoma cell line DLD-1 by 43.5 %, human colon cancer cell line HCT-116 by 36.4 %, human gastric carcinoma cell line NCI-N87 by 42.56 % and human oesophagael squamous cell carcinoma cell line KYSE-30 by 37.6 % The degree of inhibition was similar to that achieved using the known anticancer agent 5-fluororacil (5-FU), and indeed superior for the NCI-N87 cells.

### Example 4

This example describes an assessment of the anti-gastrointestinal cancer activity of 1,1-dimethylallylcaffeic acid, a compound isolated from NZ-source European-type propolis in fraction S#14 60% F8 (27-30) (Table 6, Example 2). This study was performed using proliferation assays for the human colon cancer adenocarcinoma cell line, DLD-1; human colon cancer cell line, HCT-116; human gastric carcinoma cell line, NCI-N87; and human oesophageal squamous cell carcinoma cell line, KYSE-30.

The 1,1-dimethylallylcaffeic acid used in the assays at a concentration of 200 µg/ml was a genuine standard. The same test procedure given in detail in Example 3 was also used for Example 4.

### Results

The results of the cell proliferation assays for 1,1-dimethylallylcaffeic acid are shown in Table 8. The results for the cells only (negative control), and for the 5-FU positive controls are the same as for Example 3 and so are not included in Table 8.

**Table 8. Antiproliferative activity of 1,1-dimethylallyl caffeic acid against 4 gastro-intestinal cell lines**

| Cell type | Mean OD (570nm) | SEM | P values(<0.05) | % stim. | % inhibition |
|---|---|---|---|---|---|
| DLD-1 | 0.0279 | 0.0046 | 2.50E-09 | 0 | 93.24 |
| HCT-116 | 0.0166 | 0.006 | 1.60E-05 | 0 | 96.73 |
| NCI-N87 | 0.0586 | 0.0049 | 2.40E-07 | 0 | 86.74 |
| KYSE-30 | 0.0133 | 0.0019 | 2.66E-08 | 0 | 97.8 |

### Discussion

1,1-dimethylallylcaffeic acid was extremely active against all four cancer cell lines, resulting in inhibition of proliferation of human colon adenocarcinoma cell line DLD-1 by 93.2 %, human colon cancer cell line HCT-116 by 86.7 %, human gastric carcinoma cell line NCI-N87 by 86.7 % and human oesophageal squamous cell carcinoma cell line KYSE-30 by 97.8 %.

The degree of inhibition was substantially superior to that achieved using the known anticancer agent 5-fluororacil - all cell lines except NCI-N87 were almost completely killed when outlier results are also included. The results also reconfirm the findings of Example 2, where fraction S#14 60% F8 (27-30), containing largely 1,1-dimethylallylcaffeic acid, was shown to substantially inhibit the proliferation of DLD-1.

### Example 5

This example describes an assessment of the anti-gastrointestinal cancer activity of 3-methyl-3-butenyl caffeate, a compound isolated from NZ-source European-type propolis in fraction S#15 60% F8 (35-40) (Table 6, Example 2). This study was performed using proliferation assays for the human colon cancer adenocarcinoma cell line, DLD-1; human colon cancer cell line, HCT-116; human gastric carcinoma cell line, NCI-N87; and human oesophageal squamous cell carcinoma cell line, KYSE-30.

The 3-methyl-3-butenyl caffeate used in the assays at a concentration of 200 µg/ml was chemically synthesized and identity confirmed by NMR and MS. The same test procedure given in detail in Example 3 was also used for Example 5.

### Results

The results of the cell proliferation assays for 3-methyl-3-butenyl caffeate are shown in Table 9. The results for the cells only (negative control), and for the 5-FU positive controls are the same as for Example 3 and so are not included in Table 9.

**Table 9. Antiproliferative activity of 3-methyl-3-butenyl caffeate against 4 gastro-intestinal cell lines**

| Cell type | Mean OD (570nm) | SEM | P values(<0.05) | % stim. | % inhibition |
|---|---|---|---|---|---|
| DLD-1 | 0.0345 | 0.0031 | 1.00E-11 | 0 | 91.63 |
| HCT-116 | 0.0202 | 0.0022 | 3.20E-08 | 0 | 96.03 |
| NCI-N87 | 0.0683 | 0.0093 | 6.70E-08 | 0 | 84.54 |
| KYSE-30 | 0.0222 | 0.0044 | 4.50E-07 | 0 | 96.31 |

### Discussion

3-methyl-3-butenyl caffeate was extremely active against all four cancer cell lines resulting in inhibition of proliferation of human colon adenocarcinoma cell line DLD-1 by 91.6 %, human colon cancer cell line HCT-116 by 96.0 %, human gastric carcinoma cell line NCI-N87 by 84.5 % and human oesophageal squamous cell carcinoma cell line KYSE-30 by 96.3 %.

The degree of inhibition was substantially superior to that achieved using the known anticancer agent 5-fluororacil - almost complete killing of HCT-116 and KYSE-30 cells was observed. The results also reconfirm the findings of Example 2, where impure 3-methyl-3-butenyl caffeate in fraction S#15 60% F8 (35-40) was shown to substantially inhibit the proliferation of DLD-1.

### Example 6

This example describes an assessment of the anti-gastrointestinal cancer activity of pinostrobin chalcone (used as a reference), a compound isolated from NZ-source European-type propolis in fraction S#24 60% F9 (54-57) (Table 6, Example 2). This study was performed using proliferation assays for the human colon cancer adenocarcinoma cell line, DLD-1; human colon cancer cell line, HCT-116; human gastric carcinoma cell line, NCI-N87; and human oesophageal squamous cell carcinoma cell line, KYSE-30.

The pinostrobin chalcone used in the assays at a concentration of 200 µg/ml was isolated from crude propolis and extensively purified. Its identity was confirmed by NMR and MS. The same test procedure given in detail in Example 3 was also used for Example 6.

### Results

The results of the antiproliferation assays for pinostrobin chalcone are shown in Table 10. The results for the cells only (negative control), and for the 5-FU positive controls are the same as for Example 3 and so are not included in Table 10.

**Table 10. Antiproliferative activity of pinostrobin chalcone (as a reference) against 4 gastro-intestinal cell lines**

| Cell type | Mean OD (570nm) | SEM | P values(<0.05) | % stim. | % inhibition |
|---|---|---|---|---|---|
| DLD-1 | 0.069 | 0.0031 | 2.00E-11 | 0 | 83.3 |
| HCT-116 | 0.2482 | 0.0073 | 1.30E-05 | 0 | 51.2 |
| NCI-N87 | 0.4659 | 0.015 | > 0.05 | 5.5 | 0 |
| KYSE-30 | 0.3312 | 0.013 | 1.00E-06 | 0 | 45 |

### Discussion

Pinostrobin chalcone (used as a reference) was highly to moderately active against three of the four cancer cell lines resulting in inhibition of proliferation of human colon adenocarcinoma cell line DLD-1 by 83.3 %, human colon cancer cell line HCT-116 by 51.2 %, and human oesophageal squamous cell carcinoma cell line KYSE-30 by 45.0 %.

However, pinostrobin chalcone was not active against human gastric carcinoma cell line NCI-N87. Surprisingly it was slightly but not statistically significantly stimulatory for proliferation of this cell line. The degree of inhibition for the other three cell lines was similar to or substantially superior to that achieved using the known anticancer agent 5-fluororacil. The results also reconfirm the findings of Example 2, where impure pinostrobin chalcone in fraction S#24 60% F9 (54-57) was shown to substantially inhibit the proliferation of DLD-1.

### Example 7

This example describes an assessment of the anti-gastrointestinal cancer activity of pinobanksin 3-O-acetate (used as a reference), a compound isolated from NZ-source European-type propolis in fraction S#16 60 % F8 (41-43) (Table 6, Example 2). This study was performed using proliferation assays for the human colon cancer adenocarcinoma cell line, DLD-1; human colon cancer cell line, HCT-116; human gastric carcinoma cell line, NCI-N87; and human oesophageal squamous cell carcinoma cell line, KYSE-30.

The pinobanksin 3-O-acetate used in the assays at a concentration of 200 µg/ml was a pure laboratory standard with identity confirmed by NMR and MS. The same test procedure given in detail in Example 3 was also used for Example 7.

### Results

The results of the antiproliferation assays for pinobanksin 3-O-acetate are shown in Table 11. The results for the cells only (negative control), and for the 5-FU positive controls are the same as for Example 3 and so are not included in Table 11.

**Table 11. Antiproliferative activity of pinobanksin 3-O-acetate (as a reference) against 4 gastro-intestinal cell lines**

| Cell type | Mean OD (570nm) | SEM | P values(<0.05) | % stim. | % inhibition |
|---|---|---|---|---|---|
| DLD-1 | 0.1014 | 0.0092 | 7.30E-08 | 0 | 75.4 |
| HCT-116 | 0.0477 | 0.0026 | 5.60E-08 | 0 | 90.61 |
| NCI-N87 | 0.2287 | 0.0083 | 1.00E-05 | 0 | 48.22 |
| KYSE-30 | 0.1925 | 0.0047 | 6.00E-08 | 0 | 68.01 |

### Discussion

Pinobanksin 3-O-acetate (used as a reference) was extremely to moderately active against all four cancer cell lines resulting in inhibition of proliferation of human colon adenocarcinoma cell line DLD-1 by 75.4 %, human colon cancer cell line HCT-116 by 90.6 %, human gastric carcinoma cell line NCI-N87 by 48.2 % and human oesophagael squamous cell carcinoma cell line KYSE-30 by 68.0 %.

The degree of inhibition was substantially superior to that achieved using the known anticancer agent 5-fluororacil. The results also reconfirm the findings of Example 2, where impure 3 pinobanksin 3-O-acetate in fraction S#16 60 % F8 (41-43) was shown to substantially inhibit the proliferation of DLD-1.

### Example 8

This example describes an assessment of the anti-gastrointestinal cancer activity of pinocembrin (used as a reference), a compound isolated from NZ-source European-type propolis in fraction S#18 60 % F8 (52-53) and used as a standard S#37 (Table 6, Example 2). This study was performed using proliferation assays for the human colon cancer adenocarcinoma cell line, DLD-1; human colon cancer cell line, HCT-116; human gastric carcinoma cell line, NCI-N87; and human oesophageal squamous cell carcinoma cell line, KYSE-30.

The pinocembrin used in the assays at a concentration of 200 µg/ml was a genuine standard. The same test procedure given in detail in Example 3 was also used for Example 8.

### Results

The results of the antiproliferation assays for pinocembrin are shown in Table 12. The results for the cells only (negative control), and for the 5-FU positive controls are the same as for Example 3 and so are not included in Table 12.

**Table 12. Antiproliferative activity of pinocembrin (as a reference) against 4 gastro-intestinal cell lines**

| Cell type | Mean OD (570nm) | SEM | P values(<0.05) | % stim. | % inhibition |
|---|---|---|---|---|---|
| DLD-1 | 0.0341 | 0.0021 | 1.20E-08 | 0 | 91.7 |
| HCT-116 | 0.0045 | 0.0005 | 1.40E-05 | 0 | 99.1 |
| NCI-N87 | 0.1214 | 0.0077 | 2.40E-07 | 0 | 72.5 |
| KYSE-30 | 0.0225 | 0.0034 | 2.00E-10 | 0 | 96.3 |

### Discussion

Pinocembrin (as a reference) was extremely active against all four cancer cell lines resulting in inhibition of proliferation of human colon adenocarcinoma cell line DLD-1 by 91.7 %, human colon cancer cell line HCT-116 by 99.1 %, human gastric carcinoma cell line NCI-N87 by 72.5 % and human oesophagael squamous cell carcinoma cell line KYSE-30 by 96.3 %.

The degree of inhibition was substantially superior to that achieved using the known anticancer agent 5-fluororacil, and for KYSE-30 and HCT-116 almost all cells were killed. The results also reconfirm the findings of Example 2, where both impure pinocembrin in fraction S#18 60 % F8 (52-53) and a pinocembrin standard S#37 were shown to almost completely inhibit the proliferation of DLD-1.

### Example 9

This example describes an assessment of the anti-gastrointestinal cancer activity of benzyl ferulate, a compound isolated from NZ-source European-type propolis in fraction S#23 60 % F9 (49-51) (Table 6, Example 2). This study was performed using proliferation assays for the human colon cancer adenocarcinoma cell line, DLD-1; human colon cancer cell line, HCT-116; human gastric carcinoma cell line, NCI-N87; and human oesephageal squamous cell carcinoma cell line, KYSE-30.

The benzyl ferulate used in the assays at a concentration of 200 µg/ml was chemically synthesized and its identity confirmed by NMR, MS, and by hydrolysis of the ester isolated from NZ propolis into the parent acid and alcohol which were then identified by comparison with genuine standard compounds. The same test procedure given in detail in Example 3 was also used for Example 9.

### Results

The results of the antiproliferation assays for benzyl ferulate are shown in Table 13. The results for the cells only (negative control), and for the 5-FU positive controls are the same as for Example 3 and so are not included in Table 13.

**Table 13. Antiproliferative activity of benzyl ferulate against 4 gastro-intestinal cell lines**

| Cell type | Mean OD (570nm) | SEM | P values(<0.05) | % stim. | % inhibition |
|---|---|---|---|---|---|
| DLD-1 | 0.0576 | 0.0001 | 1.50E-06 | 0 | 86.02 |
| HCT-116 | 0.0207 | 0.0027 | 1.70E-05 | 0 | 95.92 |
| NCI-N87 | 0.0576 | 0.0054 | 1.60E-05 | 0 | 86.96 |
| KYSE-30 | 0.0083 | 0.0005 | 6.50E-06 | 0 | 98.62 |

### Discussion

Benzyl ferulate was extremely active against all four cancer cell lines resulting in inhibition of proliferation of human colon adenocarcinoma cell line DLD-1 by 86.0 %, human colon cancer cell line HCT-116 by 95.9 %, human gastric carcinoma cell line NCI-N87 by 87.0 % and human oesophagael squamous cell carcinoma cell line KYSE-30 by 98.6 %.

The degree of inhibition was substantially superior to that achieved using the known anticancer agent 5-fluororacil, and for KYSE-30 and HCT-116 almost all cells were killed. The results also reconfirm the findings of Example 2, where an impure mixture of benzyl ferulate and benzyl isoferulate in fraction S#23 60 % F9 (49-51) was shown to substantially inhibit the proliferation of DLD-1.

### Example 10

This example describes an assessment of the anti-gastrointestinal cancer activity of benzyl isoferulate, a compound isolated from NZ-source European-type propolis in fraction S#23 60 % F9 (49-51) (Table 6, Example 2). This study was performed using proliferation assays for the human colon cancer adenocarcinoma cell line, DLD-1; human colon cancer cell line, HCT-116; human gastric carcinoma cell line, NCI-N87; and human oesophageal squamous cell carcinoma cell line, KYSE-30.

The benzyl isoferulate used in the assays at a concentration of 200 µg/ml was chemically synthesized and its identity confirmed by NMR, MS, and by hydrolysis of the ester isolated from NZ propolis into the parent acid and alcohol which were then identified by comparison with genuine standard compounds. The same test procedure given in detail in Example 3 was also used for Example 10.

### Results

The results of the antiproliferation assays for benzyl isoferulate are shown in Table 14. The results for the cells only (negative control), and for the 5-FU positive controls are the same as for Example 3 and so are not included in Table 14.

**Table 14. Antiproliferative activity of benzyl isoferulate against 4 gastro-intestinal cell lines**

| Cell type | Mean OD (570nm) | SEM | P values(<0.05) | % stim. | % inhibition |
|---|---|---|---|---|---|
| DLD-1 | 0.0379 | 0.0001 | 1.10E-06 | 0 | 90.81 |
| HCT-116 | 0.0264 | 0.0011 | 1.80E-05 | 0 | 94.81 |
| NCI-N87 | 0.0885 | 0.0066 | 2.80E-05 | 0 | 79.95 |
| KYSE-30 | 0.0251 | 0.0065 | 7.80E-06 | 0 | 95.82 |

### Discussion

Benzyl isoferulate was extremely active against all four cancer cell lines resulting in inhibition of proliferation of human colon adenocarcinoma cell line DLD-1 by 90.8 %, human colon cancer cell line HCT-116 by 94.8 %, human gastric carcinoma cell line NCI-N87 by 80.0 % and human oesophageal squamous cell carcinoma cell line KYSE-30 by 95.8 %.

The degree of inhibition was substantially superior to that achieved using the known anticancer agent 5-fluororacil, and almost identical to the degree of inhibition achieved with structural isomer benzyl ferulate - for KYSE-30 and HCT-116 almost all cells were killed. The results also reconfirm the findings of Example 2, where an impure mixture of benzyl ferulate and benzyl isoferulate in fraction S#23 60 % F9 (49-51) was shown to substantially inhibit the proliferation of DLD-1.

### Example 11

This example describes an assessment of the anti-gastrointestinal cancer activity of tectochrysin (used as a reference), also known as chrysin-7-methylether, a compound isolated from NZ-source European-type propolis in fraction S#27 60 % F10 and used as a comparative standard S#35 (Table 6, Example 2). This study was performed using proliferation assays for the human colon cancer adenocarcinoma cell line, DLD-1; human colon cancer cell line, HCT-116; human gastric carcinoma cell line, NCI-N87; and human oesophageal squamous cell carcinoma cell line, KYSE-30.

The tectochrysin used in the assays at a concentration of 200 µg/ml was a genuine standard. The same test procedure given in detail in Example 3 was also used for Example 11, except that the tectochrysin was dissolved in triethylene glycol mono-methyl ether, as it had not properly dissolved as sample S#35 in Example 2.

### Results

The results of the antiproliferation assays for tectochrysin are shown in Table 15. The results for the cells only (negative control), and for the 5-FU positive controls are the same as for Example 3 and so are not included in Table 15.

**Table 15. Antiproliferative activity of pinobanksin 3-O-acetate (as a reference) against 4 gastro-intestinal cell lines**

| Cell type | Mean OD (570nm) | SEM | P values(<0.05) | % stim. | % inhibition |
|---|---|---|---|---|---|
| DLD-1 | 0.0296 | 0.0016 | 9.80E-07 | 0 | 92.83 |
| HCT-116 | 0.0505 | 0.0029 | 2.60E-05 | 0 | 90.07 |
| NCI-N87 | 0.2301 | 0.0152 | 4.00E-04 | 0 | 54.01 |
| KYSE-30 | 0.0746 | 0.0042 | 8.60E-07 | 0 | 87.6 |

### Discussion

Tectochrysin (used as a reference) was extremely to moderately active against all four cancer cell lines resulting in inhibition of proliferation of human colon adenocarcinoma cell line DLD-1 by 92.8 %, human colon cancer cell line HCT-116 by 90.1 %, human gastric carcinoma cell line NCI-N87 by 54.0 % and human oesophageal squamous cell carcinoma cell line KYSE-30 by 87.6 %.

The degree of inhibition was substantially superior to that achieved using the known anticancer agent 5-fluororacil. The results also reconfirm the findings of Example 2 for S# 27 60 % F10, where impure tectochrysin was shown to substantially inhibit the proliferation of DLD-1, but differ from Example 2 S#35 as this time the sample was fully dissolved in the test medium.

### Example 12

This example describes an assessment of the anti-gastrointestinal cancer activity of para-coumaric acid (used as a reference), also known as p-coumaric acid, a compound widely associated with European-type propolis and shown to be inactive against DLD-1 when isolated from NZ propolis (data not shown in Example 2). This study was performed using proliferation assays for the human colon cancer adenocarcinoma cell line, DLD-1; human colon cancer cell line, HCT-116; human gastric carcinoma cell line, NCI-N87; and human oesophageal squamous cell carcinoma cell line, KYSE-30.

The p-coumaric acid used in the assays at a concentration of 200 µg/ml was a genuine standard.

### Results

The results of the antiproliferation assays for p-coumaric acid are shown in Table 16. The results for the cells only (negative control), and for the 5-FU positive controls are the same as for Example 3 and so are not included in Table 16.

**Table 16. Antiproliferative activity of p-coumaric acid (as a reference) against 4 gastro-intestinal cell lines**

| Cell type | Mean OD (570nm) | SEM | P values(<0.05) | % stim. | % inhibition |
|---|---|---|---|---|---|
| DLD-1 | 0.3531 | 0.003 | 6.30E-03 | 0 | 14.34 |
| HCT-116 | 0.5216 | 0.023 | > 0.05 | 2.6 | 0 |
| NCI-N87 | 0.3652 | 0.0157 | > 0.05 | 0 | 17.31 |
| KYSE-30 | 0.3243 | 0.0079 | 6.60E-05 | 0 | 46.1 |

### Discussion

p-coumaric acid (used as a reference) was moderately active against the human oesophageal squamous cell carcinoma cell line KYSE-30, resulting in inhibition of by 46.1 %. However, it was less active against the human colon adenocarcinoma cell line DLD-1, inhibiting proliferation by 14.3 %, and against the human gastric carcinoma cell line NCI-N87, inhibiting proliferation by 17.3 %, although this was not statistically significant. *p*-coumaric acid was inactive against the human colon cancer cell line HCT-116, slightly but not statistically significantly stimulating proliferation, by 2.6 %.

This compound can therefore be considered as active against the squamous cell carcinoma cell line KYSE-30.
Pincomebrin and *p*-coumaric acid were chosen as positive and negative controls on the basis of their strong and weak activity respectively.

### Example 13

This example provides a detailed assessment of the anti-oesophageal cancer activity of NZ and Polish poplar-type propolis and the most bioactive compounds isolated from propolis as tested and demonstrated in Examples 4, 5, 8 and 9 at a concentration of 200 µg/ml. This study was performed using proliferation assays for the human oesophageal squamous cell carcinoma cell line, KYSE-30 using the general method outlined in Example 3. The compounds tested were pinocembrin (as a reference) at 100 µg/ml; dimethylallyl caffeic acid at 100 µg/ml; 3-methyl-3-butenyl caffeate at 10 µg/ml; and benzyl ferulate at 50 µg/ml. The two propolis samples were a NZ propolis tincture rich in pinocembrin (as a reference sample) and pinobanksin 3-o-acetate (as a reference sample); and a Polish propolis tincture rich in p-coumaric acid. Both samples were evaporated to dryness before dissolution in the test solution to a final concentration of 50 µg/ml. The composition of the two propolis tincture samples after evaporation to dryness in mg compound per g of dry solids is given in Tables 17 and 18:

**Table 17. Flavonoid composition of propolis tinctures evaporated to dryness, mg/g dry solids**

| Propolis | pinobanksin | pinocembrin | P-3-o-A | chrysin | galangin | pino-7-me | Tecto-chrysin |
|---|---|---|---|---|---|---|---|
| NZ | 20.9 | 85.2 | 43.8 | 27.6 | 39.8 | 7.6 | 4.4 |
| Polish | 16.6 | 37.3 | 17.5 | 20.6 | 17.3 | 11.7 | 5.0 |

| | | | | | | | |
|---|---|---|---|---|---|---|---|
| P-3-o-A = pinobanksin 3-o-acetate; pino-7-me = pinocembrin-7-methyl ether | | | | | | | |

**Table 18. Phenolic acid and ester composition of propolis tinctures evaporated to dryness, mg/g dry solids**

| Propolis | Caffeic acid | *p*-coumaric | CAPE | Benzyl caffeate | Benzyl f & iso-f | 3M3B caffeate | DMA caffeate |
|---|---|---|---|---|---|---|---|
| NZ | 7.0 | 4.0 | 5.8 | 17 | 14 | 18 | 15 |
| Polish | 7.4 | 32.3 | 8.9 | 4 | 9 | 2 | 1 |

| | | | | | | | |
|---|---|---|---|---|---|---|---|
| CAPE = caffeic acid phenethyl ester; Benzyl f & iso-f = benzyl ferulate and isoferulate; 3M3B caffeate = 3-methyl-3-butenyl caffeate; DMA caffeate = dimethylallyl caffeate | | | | | | | |

NZ propolis has substantially higher levels of the flavonoids pinocembrin and pinobanksin 3-o-acetate than Polish propolis. The content of dimethylallyl caffeic acid, and 3-methyl-3-butenyl caffeate were also substantially higher, as were total levels of benzyl ferulate and benzyl isoferulate which were quantified together. The Polish propolis has substantially higher levels of p-coumaric acid, and moderately higher levels of CAPE.

The results of the KYSE-30 cell proliferation assay are shown in Table 19 for the compounds and propolis tincture samples tested, along with results for the positive control 5-fluorouracil (5-FU) at three concentrations, and the negative control (cells only with medium and no test compound or positive control). In the Table, n is the number of replicates (n < 6 indicates some replicates gave 100 % inhibition of proliferation); OD is Optical Density measured at 570 nm; SEM is the Standard Error associated with the Mean Optical Density value measured; p is the probability value that the measurement is statistically significant via the Student t-test, here taken to be < 0.05 (NS = not significant); % inhibition is the percentage reduction of proliferation compared to the negative control, with a large number indicating the test compound has anticancer proliferation potential.

**Table 19. Antiproliferative activity of test compounds against oesophageal squamous cell carcinoma cell line KYSE-30**

| Compound | Concentration | Mean OD (570nm) | SEM | P values (<0.05) | % inhibition |
|---|---|---|---|---|---|
| Cells only (n= 6) | | 0.3353 | 0.0076 | 1 | 0 |
| 5-FU (n=6) (reference) | 0.195 µg/ml | 0.3238 | 0.0202 | NS | 3.41 |
| 5-FU (n=6) (reference) | 0.65 µg/ml | 0.2795 | 0.0100 | 1.26E-03 | 16.63 |
| 5-FU (n=6) (reference) | 1.95 µg/ml | 0.2297 | 0.0052 | 4.60E-07 | 31.49 |
| NZ propolis dry solids (n=6) | 50 µg/ml | 0.1905 | 0.0084 | 1.68E-07 | 43.18 |
| Polish propolis high p-coumaric (n=6) | 50 µg/ml | 0.2360 | 0.0108 | 2.06E-05 | 29.60 |
| Pinocembrin (n=6) (reference) | 100 µg/ml | 0.1668 | 0.0081 | 3.24E-08 | 50.26 |
| Dimethylallyl caffeic acid (n=3) | 100 µg/ml | 0.0381 | 0.0065 | 4.79E-06 | 88.63 |
| Benzyl ferulate (n=5) | 50 µg/ml | 0.0398 | 0.0064 | 3.44E-10 | 88.14 |
| 3-methyl-3-butenyl caffeate (n=6) | 10 µg/ml | 0.2034 | 0.0061 | 9.72E-08 | 39.33 |

### Discussion

This Example shows that compounds isolated from propolis are more effective at inhibiting the proliferation of KYSE-30 than propolis, and thus are suitable candidate molecules for pharmaceutical preparations. This Example also provides indications of a dose response for pinocembrin (as a reference), dimethylallyl caffeic acid, benzyl ferulate and 3-methyl-3-butenyl caffeate. Both dimethylallyl caffeic acid and benzyl ferulate are demonstrated to be very highly active even at a half and a quarter or the concentration used in Examples 4 and 9 respectively. Only 3 replicates out of 6 for dimethylallyl caffeic acid gave a less than 100 % inhibition of proliferation; and only 5 replicates out of 6 for benzyl ferulate gave a less than 100 % inhibition of proliferation of KYSE-30. 3-methyl-3-butenyl caffeate is also a very strong inhibitor of proliferation, as it was still moderately active at 10 µg/ml (39.3 % inhibition), which is only 5 % of the concentration used in Example 5. Pinocembrin was still highly active (50.3 % inhibition) at 100 µg/ml. NZ propolis at 50.3 % inhibition of proliferation was more effective than Polish propolis at 29.6 % inhibition, reflecting the benefits of having high levels of the highly active dihydroflavonoids pinocembrin and pinobanksin-3-o-acetate and low levels of the moderately active p-coumaric acid.

### INDUSTRIAL APPLICABILITY

Anti-gastrointestinal cancer compositions described herein containing compounds derived from propolis or fraction thereof can be used in consumer goods including foods and beverages, medical devices, medical supplies, functional foods and pharmaceuticals. Methods of using such compositions, for example in the treatment of gastrointestinal cancers and symptoms thereof have application in the medical field.

## Claims

1. A compound of formula (II): 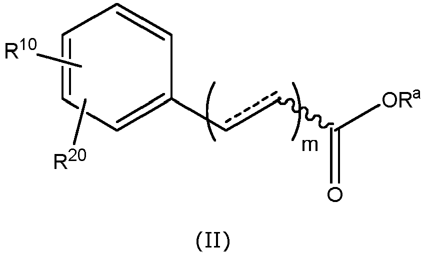 wherein:
R^{a} is C₂₋₆alkenyl or arylC₁₋₆alkyl;
m is 1 or 2; and
R¹⁰ and R²⁰ are each independently hydroxyl, or C₁₋₆alkoxy; and
provided that R^{a} is not arylC₁₋₆alkyl when R¹⁰ and R²⁰ are both hydroxyl,
for use in inhibiting gastrointestinal tumour formation, inhibiting gastrointestinal tumour growth, inhibiting gastrointestinal tumour metastasis or treating or preventing gastrointestinal cancer in a human subject; inducing apoptosis of one or more neoplastic gastrointestinal cells in a human subject; increasing the responsiveness of a human subject to a gastrointestinal cancer therapy; increasing the sensitivity of a gastrointestinal tumour in a human subject to a gastrointestinal cancer therapy; resensitising one or more gastrointestinal cancer cells in a human subject that are resistant to treatment; at least partially reversing the resistance of a neoplastic cell in a human subject suffering from gastrointestinal cancer to a gastrointestinal cancer therapy; reversing, wholly or in part, the resistance of a gastrointestinal cancer-burdened human patient to a gastrointestinal cancer therapy; or re-sensitising one or more tumours of a gastrointestinal cancer-burdened human patient which are, or are predicted to either be or become, resistant to treatment with a gastrointestinal cancer therapy to treatment with a gastrointestinal cancer therapy,
wherein the gastrointestinal tumour is a gastric tumour, and the gastrointestinal cancer is gastric cancer.

2. A composition comprising a therapeutically effective amount of a compound of formula (II): 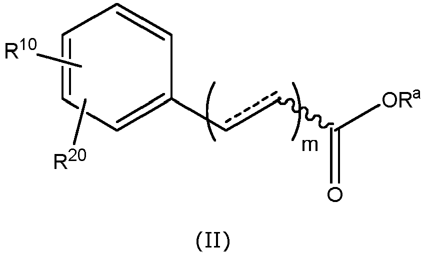 wherein:
R^{a} is C₂₋₆alkenyl or arylC₁₋₆alkyl;
m is 1 or 2; and
R¹⁰ and R²⁰ are each independently hydroxyl, or C₁₋₆alkoxy; and
provided that R^{a} is not arylC₁₋₆alkyl when R¹⁰ and R²⁰ are both hydroxyl, for use in inhibiting gastrointestinal tumour formation, inhibiting gastrointestinal tumour growth, inhibiting gastrointestinal tumour metastasis or treating or preventing gastrointestinal cancer in a human subject; inducing apoptosis of one or more neoplastic gastrointestinal cells in a human subject; increasing the responsiveness of a human subject to a gastrointestinal cancer therapy; increasing the sensitivity of a gastrointestinal tumour in a human subject to a gastrointestinal cancer therapy; resensitising one or more gastrointestinal cancer cells in a human subject that are resistant to treatment; at least partially reversing the resistance of a neoplastic cell in a human subject suffering from gastrointestinal cancer to a gastrointestinal cancer therapy; reversing, wholly or in part, the resistance of a gastrointestinal cancer-burdened human patient to a gastrointestinal cancer therapy; or re-sensitising one or more tumours of a gastrointestinal cancer-burdened human patient which are, or are predicted to either be or become, resistant to treatment with a gastrointestinal cancer therapy to treatment with a gastrointestinal cancer therapy
wherein the gastrointestinal tumour is a gastric tumour and the gastrointestinal cancer is gastric cancer.

3. The compound for use according to claim 1 or the composition for use according to claim 2, wherein the compound of formula (II) is a compound of the formula (IIA):

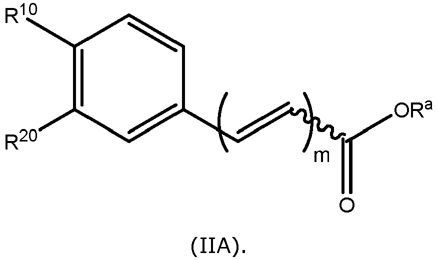

4. The compound for use according to claim 1 or 3 or the composition for use according to claim 2 or 3, wherein R¹⁰ and R²⁰ are each hydroxyl, R¹⁰ is hydroxyl and R²⁰ is C₁₋₆alkoxy, or R¹⁰ is C₁₋₆alkoxy and R²⁰ is hydroxyl.

5. The compound for use according to any one of claims 1, 3, or 4 or the composition for use according to any one of claims 2, 3, or 4, wherein the C₁₋₆alkoxy is OMe.

6. The compound for use according to any one of claims 1 or 3 to 5 or the composition for use according to any one of claims 2 or 3 to 5, wherein R^{a} is C₂₋₆alkenyl, or arylC₁₋₆alkyl.

7. The compound for use according to any one of claims 1 or 3 to 6 or the composition for use according to any one of claims 2 or 3 to 6, wherein the C₂₋₆alkenyl is prenyl or isoprenyl.

8. The compound for use according to any one of claims 1 or 3 to 6 or the composition for use according to any one of claims 2 or 3 to 6, wherein the arylC₁₋₆alkyl is benzyl.

9. The compound for use according to any one of claims 1 or 3 to 8 or the composition for use according to any one of claims 2 and 3 to 8, wherein m is 2.

10. The compound for use according to any one of claims 1 or 3 to 8 or the composition for use according to any one of claims 2 or 3 to 8, wherein m is 1.

11. The compound for use according to any one of claims 1 or 3 to 8 or 10 or the composition for use according to any one of claims 2 or 3 to 8 or 10, wherein R^{a} is C₂₋₆alkenyl, and R¹⁰ and R²⁰ are each hydroxyl, R¹⁰ is hydroxyl and R²⁰ is C₁₋₆alkoxy, or R¹⁰ is C₁₋₆alkoxy and R²⁰ is hydroxyl; or R^{a} is arylC₁₋₆alkyl, and R¹⁰ is hydroxyl and R²⁰ is C₁₋₆alkoxy, or R¹⁰ is C₁₋₆alkoxy and R²⁰ is hydroxyl.

12. The compound for use according to any one of claims 1 or 3 to 7, 10, or 11 or the composition for use according to any one of claims 2, 3 to 7, 10, or 11 wherein R^{a} is C₂₋₆alkenyl; and R¹⁰ and R²⁰ are each hydroxyl.

13. The compound for use according to any one of claims 1 or 3 to 6, 8, 10, or 11 or the composition for use according to any one of claims 2, 3 to 6, 8, 10, or 11 wherein R^{a} is arylC₁₋₆alkyl, and R¹⁰ is hydroxyl and R²⁰ is C₁₋₆alkoxy, or R¹⁰ is C₁₋₆alkoxy and R²⁰ is hydroxyl.

14. The compound for use according to claim 1 or the composition for use according to claim 2 wherein the compound is, or the composition comprises a therapeutically effective amount of at least one of, a compound selected from any one or more of
a) 1,1-dimethylallyl caffeic acid,
b) 3-methyl-3-butenyl caffeic acid,
c) benzyl isoferulate.

15. The composition for use according to any one of claims 2 to 14, wherein the composition is formulated for oral or topical administration.

## Patentansprüche

1. Verbindung der Formel(II): 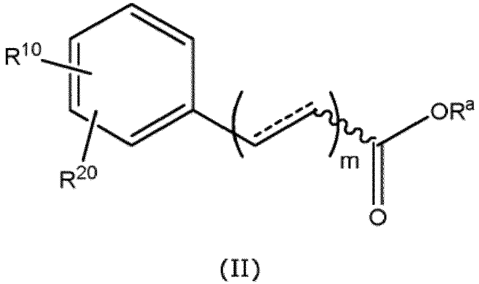 wobei:
R^{a} für C₂₋₆-Alkenyl oder Aryl-C₁₋₆-alkyl steht,
m für 1 oder 2 steht und
R¹⁰ und R²⁰ jeweils unabhängig voneinander für Hydroxyl oder C₁₋₆-Alkoxy stehen, und mit der Maßgabe, dass R^{a} nicht für Aryl-C₁₋₆-alkyl steht, wenn R¹⁰ und R²⁰ beide für Hydroxyl stehen,
zur Verwendung bei der Inhibierung der Bildung gastrointestinaler Tumore, der Inhibierung des Wachstums gastrointestinaler Tumore, der Inhibierung der Metastasenbildung gastrointestinaler Tumore oder der Behandlung oder Prävention von gastrointestinalem Krebs in einem menschlichen Subjekt, der Induktion von Apoptose einer oder mehrerer neoplastischer gastrointestinaler Zellen in einem menschlichen Subjekt, der Erhöhung des Ansprechens eines menschlichen Subjekts auf eine Therapie von gastrointestinalem Krebs, der Erhöhung der Empfindlichkeit eines gastrointestinalen Tumors in einem menschlichen Subjekt gegenüber einer Therapie von gastrointestinalem Krebs, der Resensibilisierung einer oder mehrerer gastrointestinaler Krebszellen in einem menschlichen Subjekt, die gegenüber einer Behandlung resistent sind, der zumindest teilweisen Umkehr der Resistenz einer neoplastischen Zelle in einem an gastrointestinalem Krebs leidenden menschlichen Subjekt gegenüber einer Therapie von gastrointestinalem Krebs, der vollständigen oder teilweisen Umkehr der Resistenz eines menschlichen Patienten mit gastrointestinaler Krebslast gegenüber einer Therapie von gastrointestinalem Krebs, oder der Resensibilisierung eines oder mehrerer Tumore eines menschlichen Patienten mit gastrointestinaler Krebslast, die resistent gegenüber einer Behandlung mit einer Therapie von gastrointestinalem Krebs sind oder von denen vorhergesagt wird, dass sie resistent gegenüber einer Behandlung mit einer Therapie von gastrointestinalem Krebs sind oder werden, gegenüber einer Behandlung mit einer Therapie von gastrointestinalem Krebs,
wobei es sich bei dem gastrointestinalen Tumor um einen Magentumor handelt und es sich bei dem gastrointestinalen Krebs um Magenkrebs handelt.

2. Zusammensetzung, umfassend eine therapeutisch wirksame Menge einer Verbindung der Formel (II): 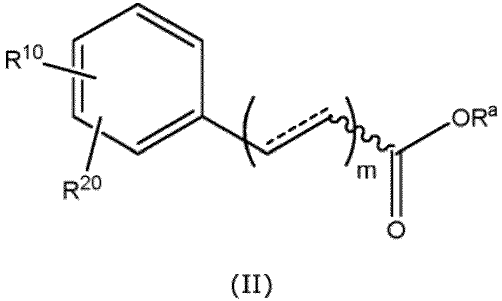 wobei:
R^{a} für C₂₋₆-Alkenyl oder Aryl-C₁₋₆-alkyl steht,
m für 1 oder 2 steht und
R¹⁰ und R²⁰ jeweils unabhängig voneinander für Hydroxyl oder C₁₋₆-Alkoxy stehen, und mit der Maßgabe, dass R^{a} nicht für Aryl-C₁₋₆-alkyl steht, wenn R¹⁰ und R²⁰ beide für Hydroxyl stehen,
zur Verwendung bei der Inhibierung der Bildung gastrointestinaler Tumore, der Inhibierung des Wachstums gastrointestinaler Tumore, der Inhibierung der Metastasenbildung gastrointestinaler Tumore oder der Behandlung oder Prävention von gastrointestinalem Krebs in einem menschlichen Subjekt, der Induktion von Apoptose einer oder mehrerer neoplastischer gastrointestinaler Zellen in einem menschlichen Subjekt, der Erhöhung des Ansprechens eines menschlichen Subjekts auf eine Therapie von gastrointestinalem Krebs, der Erhöhung der Empfindlichkeit eines gastrointestinalen Tumors in einem menschlichen Subjekt gegenüber einer Therapie von gastrointestinalem Krebs, der Resensibilisierung einer oder mehrerer gastrointestinaler Krebszellen in einem menschlichen Subjekt, die gegenüber einer Behandlung resistent sind, der zumindest teilweisen Umkehr der Resistenz einer neoplastischen Zelle in einem an gastrointestinalem Krebs leidenden menschlichen Subjekt gegenüber einer Therapie von gastrointestinalem Krebs, der vollständigen oder teilweisen Umkehr der Resistenz eines menschlichen Patienten mit gastrointestinaler Krebslast gegenüber einer Therapie von gastrointestinalem Krebs, oder der Resensibilisierung eines oder mehrerer Tumore eines menschlichen Patienten mit gastrointestinaler Krebslast, die resistent gegenüber einer Behandlung mit einer Therapie von gastrointestinalem Krebs sind oder von denen vorhergesagt wird, dass sie resistent gegenüber einer Behandlung mit einer Therapie von gastrointestinalem Krebs sind oder werden, gegenüber einer Behandlung mit einer Therapie von gastrointestinalem Krebs,
wobei es sich bei dem gastrointestinalen Tumor um einen Magentumor handelt und es sich bei dem gastrointestinalen Krebs um Magenkrebs handelt.

3. Verbindung zur Verwendung nach Anspruch 1 oder Zusammensetzung zur Verwendung nach Anspruch 2, wobei es sich bei der Verbindung der Formel (II) um eine Verbindung der Formel (IIA): 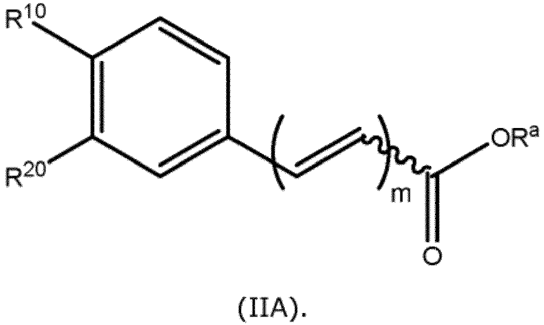 handelt.

4. Verbindung zur Verwendung nach Anspruch 1 oder 3 oder Zusammensetzung zur Verwendung nach Anspruch 2 oder 3, wobei R¹⁰ und R²⁰ jeweils für Hydroxyl stehen, R¹⁰ für Hydroxyl steht und R²⁰ für C₁₋₆-Alkoxy steht oder R¹⁰ für C₁₋₆-Alkoxy steht und R²⁰ für Hydroxyl steht.

5. Verbindung zur Verwendung nach einem der Ansprüche 1, 3 oder 4 oder Zusammensetzung zur Verwendung nach einem der Ansprüche 2, 3 oder 4, wobei C₁₋₆-Alkoxy für OMe steht.

6. Verbindung zur Verwendung nach einem der Ansprüche 1 oder 3 bis 5 oder Zusammensetzung zur Verwendung nach einem der Ansprüche 2 oder 3 bis 5, wobei R^{a} für C₂₋₆-Alkenyl oder Aryl-C₁₋₆-alkyl steht.

7. Verbindung zur Verwendung nach einem der Ansprüche 1 oder 3 bis 6 oder Zusammensetzung zur Verwendung nach einem der Ansprüche 2 oder 3 bis 6, wobei C₂₋₆-Alkenyl für Prenyl oder Isoprenyl steht.

8. Verbindung zur Verwendung nach einem der Ansprüche 1 oder 3 bis 6 oder Zusammensetzung zur Verwendung nach einem der Ansprüche 2 oder 3 bis 6, wobei Aryl-C₁₋₆-alkyl für Benzyl steht.

9. Verbindung zur Verwendung nach einem der Ansprüche 1 oder 3 bis 8 oder Zusammensetzung zur Verwendung nach einem der Ansprüche 2 und 3 bis 8, wobei m für 2 steht.

10. Verbindung zur Verwendung nach einem der Ansprüche 1 oder 3 bis 8 oder Zusammensetzung zur Verwendung nach einem der Ansprüche 2 oder 3 bis 8, wobei m für 1 steht.

11. Verbindung zur Verwendung nach einem der Ansprüche 1 oder 3 bis 8 oder 10 oder Zusammensetzung zur Verwendung nach einem der Ansprüche 2 oder 3 bis 8 oder 10, wobei R^{a} für C₂₋₆-Alkenyl steht und R¹⁰ und R²⁰ jeweils für Hydroxyl stehen, R¹⁰ für Hydroxyl steht und R²⁰ für C₁₋₆-Alkoxy steht oder R¹⁰ für C₁₋₆-Alkoxy steht und R²⁰ für Hydroxyl steht oder R^{a} für Aryl-C₁₋₆-alkyl steht und R¹⁰ für Hydroxyl steht und R²⁰ für C₁₋₆-Alkoxy steht oder R¹⁰ für C₁₋₆-Alkoxy steht und R²⁰ für Hydroxyl steht.

12. Verbindung zur Verwendung nach einem der Ansprüche 1 oder 3 bis 7, 10 oder 11 oder Zusammensetzung zur Verwendung nach einem der Ansprüche 2, 3 bis 7, 10 oder 11, wobei R^{a} für C₂₋₆-Alkenyl steht und R¹⁰ und R²⁰ jeweils für Hydroxyl stehen.

13. Verbindung zur Verwendung nach einem der Ansprüche 1 oder 3 bis 6, 8, 10 oder 11 oder Zusammensetzung zur Verwendung nach einem der Ansprüche 2, 3 bis 6, 8, 10 oder 11, wobei R^{a} für Aryl-C₁₋₆-alkyl steht und R¹⁰ für Hydroxyl steht und R²⁰ für C₁₋₆-Alkoxy steht oder R¹⁰ für C₁₋₆-Alkoxy steht und R²⁰ für Hydroxyl steht.

14. Verbindung zur Verwendung nach Anspruch 1 oder Zusammensetzung zur Verwendung nach Anspruch 2, wobei es sich bei der Verbindung um eine Verbindung ausgewählt aus einer oder mehreren von
a) 1,1-Dimethylallylkaffeesäure,
b) 3-Methyl-3-butenylkaffeesäure,
c) Isoferulasäurebenzylester
handelt bzw. die Zusammensetzung eine therapeutisch wirksame Menge davon umfasst.

15. Zusammensetzung zur Verwendung nach einem der Ansprüche 2 bis 14, wobei die Zusammensetzung für die orale oder topische Verabreichung formuliert ist.

## Revendications

1. Composé de formule (II) : 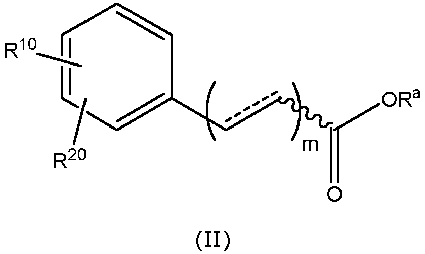 où :
R^{a} représente un groupement alcényle en C₂₋₆ ou aryl-(alkyle en C₁₋₆) ;
m est égal à 1 ou à 2 ; et
chacun des radicaux R¹⁰ et R²⁰ représente indépendamment un groupement hydroxyle ou alkoxy en C₁₋₆ ; et
à la condition que R^{a} ne représente pas un groupement aryl- (alkyle en C₁₋₆) lorsque R¹⁰ et R²⁰ représentent tous deux des groupements hydroxyle,
pour utilisation dans l'inhibition de la formation de tumeurs gastro-intestinales, l'inhibition de la croissance de tumeurs gastro-intestinales, l'inhibition des métastases des tumeurs gastro-intestinales ou le traitement prophylactique ou thérapeutique des cancers gastro-intestinaux chez un sujet humain ; l'induction de l'apoptose d'une ou de plusieurs cellules gastro-intestinales néoplasiques chez un sujet humain ;
l'augmentation de la réactivité d'un sujet humain à une thérapie contre les cancers gastro-intestinaux ;
l'augmentation de la sensibilité d'une tumeur gastro-intestinale chez un sujet humain à une thérapie contre les cancers gastro-intestinaux ; la resensibilisation d'une ou de plusieurs cellules de cancer gastro-intestinal chez un sujet humain qui sont résistantes au traitement ; l'inversion au moins partielle de la résistance d'une cellule néoplasique chez un sujet humain souffrant d'un cancer gastro-intestinal à une thérapie contre les cancers gastro-intestinaux ; l'inversion, entière ou partielle, de la résistance d'un patient humain souffrant d'un cancer gastro-intestinal à une thérapie contre les cancers gastro-intestinaux ; ou la resensibilisation d'une ou de plusieurs tumeurs d'un patient humain souffrant d'un cancer gastro-intestinal qui sont, ou dont on prévoit qu'elles sont ou qu'elles vont devenir, résistantes à un traitement par une thérapie contre les cancers gastro-intestinaux à un traitement par une thérapie contre les cancers gastro-intestinaux,
où la tumeur gastro-intestinale est une tumeur gastrique, et le cancer gastro-intestinal est un cancer gastrique.

2. Composition comprenant une quantité thérapeutiquement active d'un composé de formule (II) : 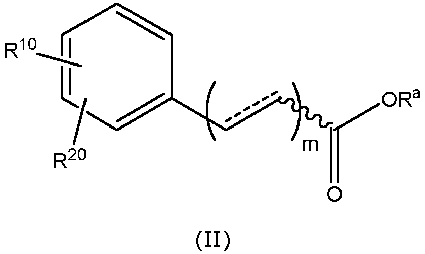 où :
R^{a} représente un groupement alcényle en C₂₋₆ ou aryl-(alkyle en C₁₋₆) ;
m est égal à 1 ou à 2 ; et
chacun des radicaux R¹⁰ et R²⁰ représente indépendamment un groupement hydroxyle ou alkoxy en C₁₋₆ ; et
à la condition que R^{a} ne représente pas un groupement aryl- (alkyle en C₁₋₆) lorsque R¹⁰ et R²⁰ représentent tous deux des groupements hydroxyle,
pour utilisation dans l'inhibition de la formation de tumeurs gastro-intestinales, l'inhibition de la croissance de tumeurs gastro-intestinales, l'inhibition des métastases des tumeurs gastro-intestinales ou le traitement prophylactique ou thérapeutique des cancers gastro-intestinaux chez un sujet humain ; l'induction de l'apoptose d'une ou de plusieurs cellules gastro-intestinales néoplasiques chez un sujet humain ; l'augmentation de la réactivité d'un sujet humain à une thérapie contre les cancers gastro-intestinaux ; l'augmentation de la sensibilité d'une tumeur gastro-intestinale chez un sujet humain à une thérapie contre les cancers gastro-intestinaux ; la resensibilisation d'une ou de plusieurs cellules de cancer gastro-intestinal chez un sujet humain qui sont résistantes au traitement ; l'inversion au moins partielle de la résistance d'une cellule néoplasique chez un sujet humain souffrant d'un cancer gastro-intestinal à une thérapie contre les cancers gastro-intestinaux ; l'inversion, entière ou partielle, de la résistance d'un patient humain souffrant d'un cancer gastro-intestinal à une thérapie contre les cancers gastro-intestinaux ; ou la resensibilisation d'une ou de plusieurs tumeurs d'un patient humain souffrant d'un cancer gastro-intestinal qui sont, ou dont on prévoit qu'elles sont ou qu'elles vont devenir, résistantes à un traitement par une thérapie contre les cancers gastro-intestinaux à un traitement par une thérapie contre les cancers gastro-intestinaux,
où la tumeur gastro-intestinale est une tumeur gastrique, et le cancer gastro-intestinal est un cancer gastrique.

3. Composé pour utilisation selon la revendication 1 ou composition pour utilisation selon la revendication 2, où le composé de formule(II) est un composé de formule (IIA) :

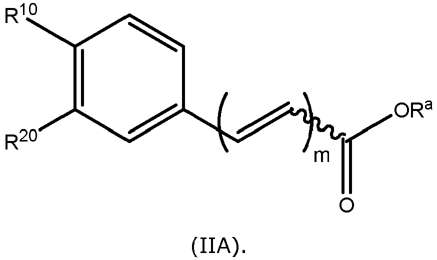

4. Composé pour utilisation selon la revendication 1 ou 3 ou composition pour utilisation selon la revendication 2 ou 3, où chacun des radicaux R¹⁰ et R²⁰ représente un groupement hydroxyle, R¹⁰ représente un groupement hydroxyle et R²⁰ représente un groupement alkoxy en C₁₋₆, ou R¹⁰ représente un groupement alkoxy en C₁₋₆ et R²⁰ représente un groupement hydroxyle.

5. Composé pour utilisation selon l'une quelconque des revendications 1, 3 ou 4 ou composition pour utilisation selon l'une quelconque des revendications 2, 3 ou 4, où le groupement alkoxy en C₁₋₆ représente OMe.

6. Composé pour utilisation selon l'une quelconque des revendications 1 ou 3 à 5 ou composition pour utilisation selon l'une quelconque des revendications 2 ou 3 à 5, où R^{a} représente un groupement alcényle en C₂₋₆ ou aryl- (alkyle en C₁₋₆).

7. Composé pour utilisation selon l'une quelconque des revendications 1 ou 3 à 6 ou composition pour utilisation selon l'une quelconque des revendications 2 ou 3 à 6, où le groupement alcényle en C₂₋₆ est un groupement prényle ou isoprényle.

8. Composé pour utilisation selon l'une quelconque des revendications 1 ou 3 à 6 ou composition pour utilisation selon l'une quelconque des revendications 2 ou 3 à 6, où le groupement aryl- (alkyle en C₁₋₆) est un groupement benzyle.

9. Composé pour utilisation selon l'une quelconque des revendications 1 ou 3 à 8 ou composition pour utilisation selon l'une quelconque des revendications 2 et 3 à 8, où m est égal à 2.

10. Composé pour utilisation selon l'une quelconque des revendications 1 ou 3 à 8 ou composition pour utilisation selon l'une quelconque des revendications 2 ou 3 à 8, où m est égal à 1.

11. Composé pour utilisation selon l'une quelconque des revendications 1 ou 3 à 8 ou 10 ou composition pour utilisation selon l'une quelconque des revendications 2 ou 3 à 8 ou 10, où R^{a} représente un groupement alcényle en C₂₋₆, et chacun des radicaux R¹⁰ et R²⁰ représente un groupement hydroxyle, R¹⁰ représente un groupement hydroxyle et R²⁰ représente un groupement alkoxy en C₁₋₆, ou R¹⁰ représente un groupement alkoxy en C₁₋₆ et R²⁰ représente un groupement hydroxyle ; ou R^{a} représente un groupement aryl-(alkyle en C₁₋₆), et R¹⁰ représente un groupement hydroxyle et R²⁰ représente un groupement alkoxy en C₁₋₆, ou R¹⁰ représente un groupement alkoxy en C₁₋₆ et R²⁰ représente un groupement hydroxyle.

12. Composé pour utilisation selon l'une quelconque des revendications 1 ou 3 à 7, 10 ou 11 ou composition pour utilisation selon l'une quelconque des revendications 2, 3 à 7, 10 ou 11, où R^{a} représente un groupement alcényle en C₂₋₆ ; et chacun des radicaux R¹⁰ et R²⁰ représente un groupement hydroxyle.

13. Composé pour utilisation selon l'une quelconque des revendications 1 ou 3 à 6, 8, 10 ou 11 ou composition pour utilisation selon l'une quelconque des revendications 2, 3 à 6, 8, 10 ou 11, où R^{a} représente un groupement aryl-(alkyle en C₁₋₆), et R¹⁰ représente un groupement hydroxyle et R²⁰ représente un groupement alkoxy en C₁₋₆, ou R¹⁰ représente un groupement alkoxy en C₁₋₆ et R²⁰ représente un groupement hydroxyle.

14. Composé pour utilisation selon la revendication 1 ou composition pour utilisation selon la revendication 2 où le composé est, ou la composition comprend une quantité thérapeutiquement active d'au moins un composé choisi parmi un ou plusieurs des suivants :
a) acide 1,1-diméthylallylcafféique,
b) acide 3-méthyl-3-buténylcafféique,
c) isoférulate de benzyle.

15. Composition pharmaceutique pour utilisation selon l'une quelconque des revendications 2 à 14, où la composition est formulée pour administration orale ou topique.
